(19) Europäisches Patentamt
European Patent Office
Office européen des brevets

(11) EP 1 965 780 B1

(12) # EUROPEAN PATENT SPECIFICATION

(45) Date of publication and mention of the grant of the patent: **08.03.2017 Bulletin 2017/10**

(21) Application number: **06842182.5**

(22) Date of filing: **19.12.2006**

(51) Int Cl.:
***A61K 31/055*** *(2006.01)* ***A61P 25/04*** *(2006.01)*
***A61P 43/00*** *(2006.01)*

(86) International application number:
**PCT/GB2006/004762**

(87) International publication number:
**WO 2007/071967 (28.06.2007 Gazette 2007/26)**

(54) **Propofol derivatives as analgesics**

Propofol-Derivate als Analgetika

Dérivés de propofol comme agents analgésiques

(84) Designated Contracting States:
**AT BE BG CH CY CZ DE DK EE ES FI FR GB GR HU IE IS IT LI LT LU LV MC NL PL PT RO SE SI SK TR**

(30) Priority: **19.12.2005 US 751559 P**
**27.04.2006 US 796270 P**

(43) Date of publication of application:
**10.09.2008 Bulletin 2008/37**

(73) Proprietors:
- **THE UNIVERSITY OF LIVERPOOL**
  **Liverpool L69 3BX (GB)**
- **The University of Dundee**
  **Dundee DD1 4HN (GB)**
- **Medizinische Hochschule Hannover**
  **30625 Hannover (DE)**

(72) Inventors:
- **LEUWER, Martin**
  **Liverpool L69 7GA (GB)**
- **HAESELER, Gertrud**
  **30625 Hannover (DE)**
- **LAMBERT, Jeremy**
  **Dundee DD1 9SY (GB)**
- **BELELLI, Delia**
  **Dundee (GB)**

(74) Representative: **Armstrong, Iain Cheshire et al**
**HGF Limited**
**4th Floor, Merchant Exchange**
**17-19 Whitworth Street West**
**Manchester M1 5WG (GB)**

(56) References cited:
**EP-A1- 0 707 849**
**WO-A-2005/033279**
**WO-A-2007/009606**
**WO-A1-00/54588**
**WO-A1-02/074200**

- **HAESLER, G ET AL.: "Structural features of phenol derivatives determining potency for activation of chloride currents via alpha-1 homomeric and alpha-1,beta heteromeric glycine receptors" BRITISH JOURNAL OF PHARMACOLOGY, vol. 145, 23 May 2005 (2005-05-23), pages 916-925, XP002429938 cited in the application**
- **HAESLER, G; LEUWER, M: "Interaction of phenol derivatives with ion channels" EUROPEAN JOURNAL OF ANAESTHESIOLOGY, vol. 19, no. 1, 2002, pages 1-8, XP009082460**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 965 780 B1

Printed by Jouve, 75001 PARIS (FR)

## Description

The present invention relates to the use of 4-bromopropofol, 4-chloropropofol and 4-iodopropofol as analgesics.

Effective safe pain control is regarded worldwide as a high priority clinical need. However the majority of developments in this field have failed to deliver high efficacy products free of undesirable side effects and safety issues. The opiates probably remain as the most effective treatment available and the ultimate goal is to deliver a pain control agent with the efficacy of the opiates but without the sedation, dependance, gastric damage and general tolerability problems.

It has been postulated that phenol derivatives may have a number of neuromodulatory effects. However the only phenol derivative in widespread clinical use is the anaesthetic propofol (2,6-di-isopropylphenol).

A published patent application (WO 00/54588) has suggested that migraine headaches, along with some other forms of pain, may be treated by propofol, by virtue of this compound's ability to act as a "rapidly-cleared anaesthetic drug".

Key features of anaesthesia are loss of consciousness, immobility in the presence of painful stimuli and absence of recall. Anaesthetics, such as propofol, are understood to mediate their anaesthetic effect by activating γ-aminobutyric acid ($GABA_A$) receptors in the Central Nervous System (CNS).

In contrast, analgesia is defined as the absence of pain. Among other peripheral and/or central nervous mechanisms, analgesia can arise as a result of enhanced inhibitory synaptic transmission within the dorsal horn of the spinal chord. It is understood that inhibitory postsynaptic transmission in the spinal chord involves mainly glycine receptors. Accordingly the glycine receptor family represents a target site for therapeutic agents aiming at inhibiting pain.

Both, $GABA_A$ and glycine receptors belong to the ligand-gated ion channel superfamily. They have a common structure in which five subunits form an ion channel, α and β subunits assemble into a pentameric receptor with a proposed *in vivo* stochiometry of 3α: 2β. Glycine receptors, like $GABA_A$ receptors, inhibit neuronal firing by opening chloride channels following agonist binding. Glycine receptors are mainly found in lower areas of the central nervous system and are involved in the control of motor rhythm generation, the coordination of spinal nociceptive reflex responses and the processing of sensory signals.

There exists a need to develop new and improved analgesics. Despite that fact that glycine receptors represent a good target for identifying such analgesics, there are no existing analgesics that target these receptors. The inventors therefore decided to address this issue and exploited their knowledge of the pathophysiological mechanisms underlying anaesthesia and analgesia with a view to identifying new and improved drugs for controlling pain.

According to the present invention there is provided a use of a compound selected from the group consisting of: 4-bromopropofol, 4-chloropropofol and 4-iodopropofol, and salts thereof; in the manufacture of a medicament for the treatment of pain.

The invention is specifically defined by the claims.

According to a first aspect of the present disclosure there is provided a use of a compound of general formula I:

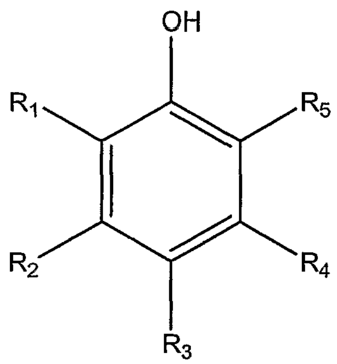

(I)

wherein $R_3$ is a halogen, amine or amide;

wherein at least one of $R_1$, $R_2$, $R_4$ and $R_5$ is an alkyl comprising between 2 and 13 carbon atoms and the others are independently selected from H or an alkyl comprising between 2 and 13 carbon atoms;

and salts thereof;

in the manufacture of a medicament for the treatment of pain.

According to second aspect of the present disclosure there is provided a method of treating pain in a subject

in need of such treatment comprising administering to said subject a therapeutically effective amount of a compound of general formula I.

According to third aspect of the disclosure there is provided a compound as defined by the first aspect of the invention for use as a medicament.

The inventors recognized that a loss of inhibitory synaptic transmission within the dorsal horn of the spinal cord plays a key role in the development of chronic pain following inflammation or nerve injury. Furthermore they recognized that inhibitory postsynaptic transmission in the spinal cord involves mainly glycine. This lead them to realise that the strychnine-sensitive glycine receptor family represents a target site for therapeutic agents aiming at inhibiting pain sensitization. This realization was based upon work conducted by Ahmadi et al. (Nature Neuroscience (2002 ) Vol. 5 No.1 p34-40).

The inventors proceeded to test their hypothesis by studying phenol derivatives with a halogen in the *para* position to the hydroxyl group and one or two methyl groups in the *ortho* or *meso* positions. Their results were published by Haeseler et al. (British Journal of Pharmacology (2005) 145, p916-925) and established that halogenation improved co-activation or activation of glycine receptors. However these initial results appeared to demonstrate that the number or position of the methyl groups on the phenol ring did not significantly effect the $EC_{50}$ for co-activation of the glycine receptors.

The inventors therefore endeavoured to develop improved analgesics that specifically target glycine receptors and to their surprise, they discovered that compounds according to the first aspect of the invention, wherein the alkyl group comprises 2 or more carbon atoms, have unexpected potency as co-activators of glycine receptors. It is preferred that compounds according to the first aspect of the invention are ligands for strychnine-sensitive glycine receptors. It is more preferred that the compounds bind to strychnine-sensitive glycine receptors with more specificity than GABA receptor types in the CNS. The inventors proceeded by making derivatives of the anaesthetic propofol and were surprised to find that compounds of general formula I are extremely potent positive allosteric modulators of strychnine sensitive glycine receptors.

The inventors used propofol as a starting point because they recognized that the glycine receptor $\alpha 1$ subunit shares primary sequence homology with transmembrane segments of $\alpha$, $\beta$ and $\gamma$ subunits of the $GABA_A$ receptor in a region of the subunit that harbours amino acid residues crucial for the binding of alcohols, volatile anaesthetics and propofol. Furthermore they recognized that propofol had been reported to activate glycine receptors at 10-fold higher concentrations than its $EC_{50}$ at $GABA_A$ receptors. As this means that the desired glycine activating effect of propofol would only be available at concentrations that in clinical practice would induce deep coma, they decided to generate other derivatives of propofol to investigate whether or not such derivatives would exhibit improved activity at glycine receptors and thereby be useful as analgesics.

The inventors were surprised to find that compounds of general formula I were extremely potent at co-activating glycine receptors and were therefore useful as analgesics. They believe that the introduction of: (1) $R_3$ at the *para* position of phenol; and (2) the introduction of the specified alkyl or alkylene-containing groups results in phenol derivatives with $EC_{50}$ values for co-activation of Glycine receptors that were significantly (by up to three orders of magnitude) lower than the most potent compounds known to the art. The compounds described in Examples 2-4 showed half-maximum potentiating effects in the low $\mu$M range or even nanomolar range. This represents more than 20-fold lower concentrations than for propofol. Furthemore compounds according to the invention were significantly more potent that the methylated phenol derivatives described in Example 1. These methylated phenols represent useful analgesics with $EC_{50}$ values for co-activation of Glycine receptors in the low $\mu$M range. However preferred compounds according to the invention, which also unexpectedly show good selectivity for glycine receptor activation over GABA receptors, have $EC_{50}$ values in the nanomolar range. This is a great advantage because it means that compounds according to the invention will be ideal analgesics and will have no or negligible effects on consciousness (i.e. anaesthetic effects).

It is preferred that the compounds have selectivity for strychnine-sensitive glycine receptors over $GABA_A$ receptors. The compounds may have an $EC_{50}$ for co-activating glycine receptors at a lower concentration than their $EC_{50}$ at $GABA_A$ receptors. Prefererably the compounds have an $EC_{50}$ for co-activating glycine receptors that is 10-fold lower than its $EC_{50}$ at $GABA_A$ receptors. It is more preferred that the compounds have an $EC_{50}$ for co-activating glycine receptors that is at least 100-fold lower than its $EC_{50}$ at $GABA_A$ receptors.

The compounds should also have an $EC_{50}$ for co-activating glycine receptors that is lower than that of propofol. For instance the compound may have an $EC_{50}$ for co-activating glycine receptors that is at least 10-fold lower or 100-fold lower than that of propofol. Most preferred compounds (e.g. 2,6 di-isopropyl-4-chlorophenol) have an $EC_{50}$ for co-activating glycine receptors that is 1000-fold lower than that of propofol (measured on glycine receptors heterologously expressed in HEK293 cells).

Suitable methods for measuring $EC_{50}$ values for co-activating glycine receptors are disclosed in Example 1.

The efficacy of the compounds is all the more surprising when the neurophysiology modulating anaesthesia in the CNS and analgesia in the PNS is considered. The inventors believe that compounds according to general formula I act as positive allosteric modulators at strychnine-sensitive glycine receptors. These receptors are chloride channels

that stabilise membrane potential by hyperpolarisation and constitute the predominant inhibitory principle at the spinal cord level. In contrast, the closely related $GABA_A$ receptor constitutes the predominant inhibitory principle in the CNS. A $GABA_A$ agonistic drug will, therefore, lead to an alteration or a loss of consciousness, whereas a compound according to the invention will ideally block pain at the peripheral level at concentrations that will not affect consciousness. The inventors believe compounds according to the invention have efficacy because they act as positive allosteric modulators at strychnine-sensitive glycine receptors and thereby block centripetal nerve signals at the dorsal root ganglionic level but have minimal or no effects at central $GABA_A$ receptors. It therefore follows, that a skilled person would choose an analgesic that was a glycine receptor agonist and which would have no $GABA_A$ agonistic effect at all. Consequently, propofol, which is the most potent $GABA_A$ agonist known, would be regarded by a skilled person as the least suitable compound to serve as a platform for developing analgesics. Furthermore a skilled person would also have reviewed the data published by Haeseler *et al.* (*supra*) and would have come to the view that the nature of the alkylation (methyl groups in the Haeseler paper) would not have been critical when developing a compound that is selective for glycine receptors over GABA receptors. The inventors therefore believe that there was a technical prejudice against investigating the analgesic properties of propofol derivatives. Thus, the extra-ordinary increase in glycine receptor co-activation that the inventors found with compounds according to the invention was not only surprising but would have been considered unlikely by the skilled artisan. In theory, any other phenol derivative with a lesser potency at the $GABA_A$ receptor level should, according to the state of the art, have been considered to be a more promising candidate.

It is preferred that $R_3$ is a halogen. The halogen may be Fluorine, Chlorine, Iodine or Bromine. The results presented in Example 2 demonstrate that unexpectedly good efficacy is exhibited by compounds falling within the scope of the present invention that are substituted at the 4-position with any desirable halogen, particularly chlorine, bromine or iodine. These results support the proposed physiological mechanism, i.e. positive modulation of the effect of a sub-maximal concentration of the natural transmitter glycine.

When conducting experiments on derivatives of propofol the inventors established that certain preferred compounds (e.g. the 4-chloro-, 4-bromo- and 4-iodo-derivatives of 2,6-di-isopropylphenol tested in Example 2) exhibited very low $EC_{50}$ values in the single-digit nanomolar range. This led the inventors to realise that the nature of $R_1$, $R_2$, $R_4$ or $R_5$ was surprisingly critical when it came to potency at glycine receptors (and therefore usefulness as an analgesic) but without unduly influencing GABA receptors and thereby effecting consciousness.

It is preferred that two of $R_1$, $R_2$, $R_4$ and $R_5$ are H and the other two are an alkyl comprising between 2 and 13, more preferably between 3 and 6 carbon atoms. It is more preferred that the two alkyl groups are both in the ortho or meso positions, respectively. Accordingly it is most preferred that $R_1$ and $R_5$ are alkyl groups and $R_2$ and $R_4$ are hydrogen or alternatively $R_2$ and $R_4$ are alkyl groups and $R_1$ and $R_5$ are hydrogen. Preferably $R_1$, $R_2$, $R_4$ and $R_5$ are each separately a hydrogen atom or a straight or branched substituted or unsubstituted alkyl group, such as an ethyl group, n-propyl, iso-propyl group, n-butyl group or an iso-butyl group. Most preferably two of $R_1$, $R_2$, $R_4$ and $R_5$ are iso-propyl groups, preferably with the remaining two of $R_1$, $R_2$, $R_4$ and $R_5$ being hydrogen atoms. Examples of such preferred compounds for use according to the invention include: 2,6-di-isopropyl-4-chlorophenol, 2,6-di-isopropyl-4-iodophenol, 2,6-di-isopropyl-4-fluorophenol, 2,6-di-isopropyl-4-bromophenol, 3,5-di-isopropyl-4-chlorophenol, 3,5-di-isopropyl-4-iodophenol, 3,5-di-isopropyl-4-flurophenol and 3,5-di-isopropyl-4-bromo-phenol.

2,6-di-isopropyl-4-chlorophenol, 2,6-di-isopropyl-4-bromophenol, and 2,6-di-isopropyl-4-iodophenol are the particularly preferred compounds for use according to the invention.

The inventors have demonstrated (see the examples) that each of these compounds surprisingly enhances the function of glycine receptors heterologously expressed in HEK293 cells at 1000-fold lower concentrations than the parent compound propofol. A skilled person will appreciate that this makes these compounds particularly useful as an analgesic as described herein.

Compounds according to the invention and medicaments containing such compounds may be used as analgesics in a number of circumstances.

The compounds are particularly useful for targeting chronic pain states (e.g. neuropathic and/or post-inflammatory chronic pain) that, so far, have been notoriously difficult to treat. The compounds are particularly useful for treating chronic neuropathic pain which is hard to treat with conventional drugs such as NSAIDs, opiate deriatives etc

The compounds are also useful for treating acute pain (e.g. following injury).

The compounds of the invention are also beneficial because they avoid all the familiar side effects of local anaesthetics and analgesics as well as NSAIDs and opioids if used as a monotherapy while, at the same time, allowing a vast variety of combined treatment strategies aiming at additive or supra-additive effects.

Examples of specific conditions in which pain may be modulated include chronic lower back pain, arthritis, cancer pain, trigeminal neuralgia, stroke and neuropathic pain.

The compounds may be used to treat existing pain but may also be used when prophylactic treatment is considered medically necessary, for instance, in advance of elective surgery.

The compounds may be used as an analgesic in the form of a monotherapy (*i.e.,* use of the compound alone) or alternatively the compounds may be given in combination with other treatments that also reduce pain. Preferred

combination therapy involves the use of the compounds with analgesics that modulate pain by a pain processing pathway that is different to pathway modulated by compounds of general formula. Such analgesics include morphine, paracetamol, and NSAIDS. The compounds may also be usefully combined with local anaesthetics (e.g. lignocaine) that only indirectly interact with glycine receptors.

The medicaments of the invention may comprise a compound of general formula I and a pharmaceutically acceptable vehicle. It will be appreciated that the vehicle should be one which is well tolerated by the subject to whom it is given and enables delivery of the compounds to the affected area.

The medicaments of the invention may take a number of different forms depending, in particular on the manner in which the compound is to be used. Thus, for example, the medicament may comprise a compound in the form of a salt of the phenol derivative (e.g. a sodium salt). Such salts may be manufactured in a powder form and incorporated in a tablet, capsule, liquid, ointment, cream, gel, hydrogel, aerosol, spray, micelle, transdermal patch, liposome or any other suitable form that may be administered to a person or animal.

Alternatively the phenol derivative according to the invention may be dissolved in a suitable solvent to form a liquid medicament. The solvent may be aqueous (e.g. PBS or distilled water). Alternatively the solvent may be an alcohol such as ethanol or a mixture of such a solvent with an aqueous solvent.

It is preferred that the medicament is used for topical or local treatment. Such medicaments may be formulated as a liquid for application to an effected site. Alternatively the liquid may be formulated for administration by injection or as an aerosol.

The compound may also be incorporated within a slow or delayed release device. Such devices may, for example, be inserted on or under the skin and the compound may be released over weeks or even months. Such a device may be particularly useful for patients with long-term chronic pain (e.g. a patient with arthritis). The devices may be particularly advantageous when a compound is used which would normally require frequent administration.

It will be appreciated that the amount of a compound required is determined by biological activity and bioavailability which in turn depends on the mode of administration, the physicochemical properties of the compound employed and whether the compound is being used as a monotherapy or in a combined therapy. The frequency of administration will also be influenced by the abovementioned factors and particularly the half-life of the compound within the subject being treated.

Optimal dosages to be administered may be determined by those skilled in the art, and will vary with the particular compound in use, the strength of the preparation, the mode of administration, and the extent of the pain requiring relief. Additional factors depending on the particular subject being treated will result in a need to adjust dosages, including subject age, weight, gender, diet, and time of administration.

Known procedures, such as those conventionally employed by the pharmaceutical industry (e.g. *in vivo* experimentation, clinical trials, etc.), may be used to establish specific formulations of compositions and precise therapeutic regimes (such as daily doses of the compounds and the frequency of administration).

Generally, a dose should be given that is effective for delivering a compound at the target site such that the tissue concentration is around the $EC_{50}$ of the compound used.

Daily doses may be given as a single administration (e.g. as a single daily injection). Alternatively, the compound used may require administration twice or more times during a day. As an example, 2,6-di-isopropyl-4-chlorophenol, for treating chronic lower back pain may be administered as two (or more depending upon the severity of the pain) daily doses of an injectable solution or an ointment. A patient receiving treatment may take a first dose upon waking and then a second dose in the evening (if on a two dose regime) or at 3 or 4 hourly intervals thereafter. Alternatively, a slow release device may be used to provide optimal doses to a patient without the need to administer repeated doses.

This invention further provides a pharmaceutical composition comprising a therapeutically effective amount of the compound of the invention and a pharmaceutically acceptable vehicle. In one embodiment, the amount of a salt of a phenol derivative (e.g. 2,6-di-isopropyl-4-chlorophenol) is an amount from about 10μg/kg Body Weight to 10mg/kg Body Weight in each dose unit for enteral (oral, rectal) administration. In another embodiment, the amount is from about 1μg/kg Body Weight to 1mg/kg Body Weight in each dose unit for parenteral(intravenous/intrathecal or epidural) administration.

In a further embodiment, the vehicle is a liquid and the composition is a solution. Useful liquid solutions for parenteral administration may comprise between 0.001 and 1 % by weight of the phenols of formula I. In another embodiment, the vehicle is a solid and the composition is a tablet. In a further embodiment, the vehicle is a gel and the composition is for topical application.

In the subject invention a "therapeutically effective amount" is any amount of a compound, medicament or composition which, when administered to a subject suffering from a painful condition against which the compounds are effective, causes reduction, remission, or regression of the pain.

A "subject" is a vertebrate, mammal, domestic animal or human being.

In the practice of this invention the "pharmaceutically acceptable vehicle" is any physiological vehicle known to those of ordinary skill in the art useful in formulating pharmaceutical compositions. In one embodiment, the pharmaceutical vehicle may be a liquid and the pharmaceutical composition would be in the form of a solution. In another embodiment, the pharmaceutically acceptable vehicle is a solid and the composition is in the form of a powder or tablet. In a further embodiment, the pharmaceutical vehicle is a gel and the composition is in the form of a suppository or cream. In a further embodiment the compound or composition may be formulated as a part of a pharmaceutically acceptable transdermal patch.

A solid vehicle can include one or more substances which may also act as lubricants, solubilizers, suspending agents, fillers, glidants, compression aids, binders or tablet-disintegrating agents; it can also be an encapsulating material. In powders, the vehicle is a finely divided solid which is in admixture with the finely divided active ingredient. In tablets, the active ingredient is mixed with a vehicle having the necessary compression properties in suitable proportions and compacted in the shape and size desired. The powders and tablets preferably contain up to 99% of the active ingredient. Suitable solid vehicles include, for example, calcium phosphate, magnesium stearate, talc, sugars, lactose, dextrin, starch, gelatin, cellulose, polyvinylpyrrolidine, low melting waxes and ion exchange resins.

Liquid vehicles are used in preparing solutions, suspensions, emulsions and the like. The phenol derivative can be dissolved or suspended in a pharmaceutically acceptable liquid vehicle such as water, ethanol, an organic solvent or mixtures thereof or pharmaceutically acceptable oils or fats.

Liquid pharmaceutical compositions which are sterile solutions or suspensions can be utilized by for example, intramuscular, intrathecal, epidural, intraperitoneal or subcutaneous injection. Sterile solutions can also be administered intravenously. The compounds may be prepared as a sterile solid composition which may be dissolved or suspended at the time of administration using sterile water, saline, or other appropriate sterile injectable medium.

The disclosure also provides a method of screening a compound for efficacy as an analgesic, the method comprising applying a test compound to a tadpole and monitoring the effect of the compound on GABA neurotransmission mediated behaviour and monitoring the effect of the compound on glycine neurotransmission mediated behaviour wherein a compound that exclusively or mostly induces behaviour characteristic of glycine neurotransmission is a putative analgesic.

Preferred aspects of the screening method are disclosed in Example 3.

The invention will be illustrated further by Examples and with reference to the following drawings, in which:

**Figure 1:** illustrates the chemical structures of the phenol derivatives tested in Example 1 from top to bottom: the compounds with the methyl groups in ortho position to the phenolic hydroxyl group; the compound with a single methyl group in meso position and its halogenated analogue; and the compound with two methyl groups in meso position and its halogenated analogue.

**Figure 2: A:** Representative current traces elicited by 2 s application of 3,5-dimethylphenol or 2,6-dimethylphenol with respect to the current elicited by 1000 $\mu$M glycine in the same experiment (upper trace). Tracings were obtained from one HEK 293 cell each expressing either $\alpha_1$ homomeric or $\alpha_1\beta$ glycine receptors. **B:** Normalized $Cl^-$ currents activated in the absence of glycine via $\alpha_1$ homomeric (triangles) or $\alpha_1\beta$ (circles) glycine receptors (mean $\pm$ SD; n = 3 each), plotted against the concentration of 3,5 dimethylphenol (upper diagram) or 2,6 dimethylphenol (lower diagram) on a logarithmic scale. Currents were normalized either to maximum value achieved by high concentrations (3000 $\mu$M) of the compound (filled symbols) or to maximum value achieved by 1000 $\mu$M glycine (empty symbols). Solid lines are Hill fits to the data with the indicated parameters. The concentration-response plots were almost superimposable for $\alpha_1$ homomeric and $\alpha_1\beta$ glycine receptors, and no difference between the ortho- and meso-methylated compound could be detected; all as discussed in Example 1.

**Figure 3:** Normalized $Cl^-$ currents activated by glycine via $\alpha_1$ homomeric (triangles) or $\alpha_1\beta$ (circles) glycine receptors (mean $\pm$ SD; n = 3 each), plotted against the concentration of glycine as referred to in Example 1. Solid lines are Hill fits to the data with the indicated parameters.

**Figure 4:** Representative current traces elicited by 2 s co-application of 10 $\mu$M glycine and (from left to right) 2-methylphenol, 3-methylphenol, and 3-methyl-4-chlorophenol with respect to the current elicited by 1000 $\mu$M glycine in the respective control experiment (upper trace) in $\alpha_1$ß heteromeric receptors as described in Example 1. All compounds increased the amplitude of the response evoked by 10 $\mu$M glycine. In the halogenated compound (right row of traces) this effect was observed in the low $\mu$M concentration range.

**Figure 5:** Representative current traces elicited by 2 s co-application of 10 $\mu$M glycine and (from left to right) 2,6 dimethylphenol, 3,5 dimethylphenol, and 3,5 dimethyl-4-chlorophenol with respect to the current elicited by 1000 $\mu$M glycine in the respective control experiment (upper trace) in $\alpha_1$ß heteromeric receptors as described in Example 1. The halogenated compound (right row of traces) showed co-activating effects in the low $\mu$M concentration range.

**Figure 6:** Representative current traces elicited via $\alpha_1$ homomeric receptors by 2 s co-application of 10 $\mu$M glycine with either 3,5 dimethylphenol (upper row of traces), 3 methylphenol (lower row of traces) or their respective halogenated analogue (right row of traces) with respect to the current elicited by 1000 $\mu$M glycine (upper trace) as described in Example 1. The effect elicited by 10 $\mu$M glycine is higher in $\alpha_1$ homomeric receptors than in $\alpha_1$ß heteromeric receptors (compare with tracings in Figs 3 and 4). Co-activating effects of phenol derivatives in $\alpha_1$ homomeric receptors are seen in a similar concentration range compared to $\alpha_1$ß heteromeric receptors.

**Figure 7:** Potentiation (%) of the current elicited by 10 $\mu$M glycine (mean $\pm$ SD of 5-6 independent experiments) by each compound in $\alpha_1$ß heteromeric receptors, plotted against the concentration applied on a logarithmic scale as described in Example 1. Solid lines are Hill fits to the data with the parameters indicated in **Table 1.** The concentrations required for a half-maximum co-activating response were significantly smaller in the halogenated compounds compared with their non-halogenated structural analogues ($p<0.0001$). No significant differences between the compounds were detected with respect to the degree of maximum potentiation. Only the potentiating effect seen with 3 methylphenol was higher than with 2 methylphenol ($p = 0.04$), which, however, might be a consequence of the lower response to glycine 10 $\mu$M in the experiments with 3 methylphenol with respect to the experiments with 2 methylphenol.

**Figure 8:** Inhibitory effects induced by 3 methyl-4-chlorophenol (left row of traces) and 3,5 dimethyl-4-chlorophenol (right row of traces) at concentrations $\geq$ 600 and 300 $\mu$M, respectively, as revealed by a reduction in the peak current amplitude during co-application with glycine 1000 $\mu$M, a concentration-dependent acceleration of the current decay during application followed by channel reopening at the end of the application as described in Example 1.

**Figure 9:** Representative current traces, as discussed in Example 2, showing co-activation of the current response to 10 $\mu$M glycine when 4-chloropropofol was co-applied with 10 $\mu$M glycine (3rd, 4th, 5th and 6th current trace from top). The first trace shows current elicited by a supramaximal glycine concentration (1000 $\mu$M).

**Figure 10:** Concentration-dependence of the coactivation of the current response to 10 $\mu$M glycine by 4-chloropropofol (mean / SD, n = 6) as discussed in Example 2.

**Figure 11:** Concentration-dependence of the coactivation of the current response to 10 $\mu$M glycine by 4-chloropropofol (mean / SD, n = 5) as discussed in Example 2. The solid line is a Hill fit to the data with the indicated parameters.

**Figure 12:** Representative current traces, as discussed in Example 2, elicited by 4-chloropropofol in the absence of the natural agonist glycine (2nd, 3rd and 4th trace from top) with respect to the current elicited by a supramaximal glycine concentration (1000 $\mu$M), top.

**Figure 13:** Normalized $Cl^-$ currents (mean $\pm$ SD, n=4) activated by 4-chloropropofol normalized to its own maximum response (empty symbols) or the maximum response elicited by 1000 $\mu$M glycine (filled symbols) plotted against the concentration of glycine as referred to in Example 2. Solid line is Hill fit to the data with the indicated parameters.

**Figure 14:** Representative current traces, as discussed in Example 2, showing co-activation of the current response to 10 $\mu$M glycine when 4-bromopropofol was co-applied with 10 $\mu$M glycine (3rd, 4th, 5th and 6th current trace from top). The first trace shows current elicited by a supramaximal glycine concentration (1000 $\mu$M).

**Figure 15:** Concentration-dependence of the coactivation of the current response to 10 $\mu$M glycine by 4-bromopropofol (mean / SD, n = 5) as discussed in Example 2. The solid line is a Hill fit to the data with the indicated parameters.

**Figure 16:** Representative current traces, as discussed in Example 2, elicited by 4-bromopropofol in the absence of the natural agonist glycine (2nd, 3rd and 4th trace from top) with respect to the current elicited by a supramaximal glycine concentration (1000 $\mu$M), top.

**Figure 17:** Normalized $Cl^-$ currents (mean $\pm$ SD, n=4) activated by 4-bromopropofol normalized to its own maximum response (empty symbols) or the maximum response elicited by 1000 $\mu$M glycine (filled symbols) plotted against the concentration of glycine as referred to in Example 2. Solid line is Hill fit to the data with the indicated parameters.

**Figure 18:** Representative current traces, as discussed in Example 2, showing co-activation of the current response to 10 $\mu$M glycine when 4-iodopropofol was co-applied with 10 $\mu$M glycine (3rd, 4th, 5th and 6th current trace from top). The first trace shows current elicited by a supramaximal glycine concentration (1000 $\mu$M).

**Figure 19:** Concentration-dependence of the coactivation of the current response to 10 μM glycine by 4-iodopropofol (mean / SD, n = 4) as discussed in Example 2. The solid line is a Hill fit to the data with the indicated parameters.

**Figure 20:** Representative current traces, as discussed in Example 2, elicited by 4-iodopropofol in the absence of the natural agonist glycine (2nd, 3rd and 4th trace from top) with respect to the current elicited by a supramaximal glycine concentration (1000 μM), top.

**Figure 21:** Normalized $Cl^-$ currents (mean ± SD, n=4) activated by 4-iodopropofol normalized to its own maximum response (empty symbols) or the maximum response elicited by 1000 μM glycine (filled symbols) plotted against the concentration of glycine as referred to in Example 2. Solid line is Hill fit to the data with the indicated parameters.

**Figure 22:** (A)illustrates tadpoles used according to Example 3; (B)is an illustrative trace for fictive swimming recorded at rostral, contra and caudal positions.

**Figure 23:** illustrates the effect of 4 chloropropofol on fictive swimming in tadpoles as discussed in Example 3 at 3.3.1.

**Figure 24:** represents tabulated data discussed in Example 3 at 3.3.1.

**Figure 25:** illustrates the effect of 4 chloropropofol and bicuculline methiodide on ventral root activity in tadpoles as discussed in Example 3 at 3.3.2.

**Figure 26:** represents tabulated data discussed in Example 3 at 3.3.2.

**Figure 27:** illustrates the effect of 4 chloropropofol and strychnine on ventral root activity in tadpoles as discussed in Example 3 at 3.3.3.

## EXAMPLE 1

This Example illustrates preliminary experiments (some of which were published in Haeseler *et al.* (supra) that were conducted to investigate the effect of a number of phenol derivatives on Glycine receptor activation and chloride currents. A skilled person will appreciate that these data demonstrate that halogenation at the *para* position of phenol improves activation of glycine receptors and suggests the compounds are suitable for use as analgesics. However these data do not anticipate the surprising extra influence that the nature of the alkylation of the phenol ring may have on the suitability of a compound on analgesic activity. This is discussed in Example 2.

### 1.1 Methods

*1.1.1 Cell culture, transfection*

Rat $\alpha_1$ and $\alpha_1\beta$ glycine receptor subunits were transiently transfected into transformed human embryonic kidney cells (HEK 293). α1 glycine receptor subunits efficiently form homomeric receptors in heterologous expression systems. ß subunits do not form homomeric receptors but affect the function of heteromeric receptors, i.e. decreasing the sensitivity to the agonistic effect of glycine and to the blocking effects of picrotoxin analogues. When co-transfecting the glycine receptor α and ß subunits, their respective cDNAs were combined in a ratio of 1:10, since expression of the ß polypeptide is less efficient than that of the α subunits. Reduced sensitivity to 1000 μM picrotoxin in $\alpha_1\beta$ heteromeric receptors was used as an assay of the efficacy of ß subunit expression. Cells were cultured in Dulbecco's modified Eagle's medium (DMEM, Biochrom, Berlin, Germany), supplemented with 10% fetal calf serum (FCS, Biochrom, Berlin, Germany), 100 U $ml^{-1}$ penicillin and 100 μg $ml^{-1}$ streptomycin at 37° C in a 5% $CO_2$ / air incubator. For transfection, cells were suspended in a buffer containing 50 mM $K_2HPO_4$ and 20 mM K-acetate, pH 7.35. For co-transfection of rat $\alpha_1$ and β glycine receptor subunits, the corresponding cDNA, each subcloned in the pCIS2 expression vector (Invitrogen, San Diego, USA) was added to the suspension. To visualize transfected cells, they were co-transfected with cDNA of green fluorescent protein (GFP 10 μg $ml^{-1}$). For transfection, we used an electroporation device by EquiBio (Kent, UK). Transfected cells were replated on glass-coverslips and incubated 15-24 h before recording.

*1.1.2 Chemicals & Solutions*

All chemicals were from Sigma Chemicals (Deisenhofen, Germany), unless otherwise noted.

The phenol derivatives under investigation were prepared as 1 M stock solution in ethanol, light-protected and

stored in glass vessels at -20 °C. Concentrations were calculated from the amount injected into the glass vials. Drug-containing vials were vigorously vortexed for 60 min. Glycine and picrotoxin were dissolved directly in bath solution.

Patch electrodes contained [mM] KCl 140, $MgCl_2$ 2, EGTA 11, HEPES 10, glucose 10; the bath solution contained [mM] NaCl 162, KCl 5.3, $NaHPO_4$ 0.6, $KH_2PO_4$ 0.22, HEPES 15, glucose 5.6.

*1.1.3 Experimental set-up*

Standard whole-cell experiments (Hamill et al., (1981) Pflügers Arch., 391, 85-100.) were performed at -30 mV membrane potential. A tight electrical seal of several GΩ formed between the cell membrane and a patch-clamp electrode allows inward currents due to agonist-induced channel activation to resolve in the pA range. Electrical resistance of the pipettes was around 5 MΩ, corresponding to a total access resistance in the whole-cell configuration of about 10 MΩ. An ultra-fast liquid filament switch technique (Franke et al., (1987) Neurosci. Lett., 77, 199-204) was used for the application of the agonist in pulses of 2 s duration. The agonist and/or the drug under investigation were applied to the cells via a smooth liquid filament achieved with a single outflow (glass tubing 0.15 mm inner diameter) connected to a piezo crystal. The cells were placed at the interface between this filament and the continuously flowing background solution. When a voltage pulse was applied to the piezo, the tube was moved up and down onto or away from the cell under investigation. Correct positioning of the cell in respect to the liquid filament was ensured applying a saturating (1000 μM) glycine pulse before and after each test experiment. Care was taken to ensure that the amplitude and the shape of the glycine-activated current had stabilized before proceeding with the experiment. Test solution and glycine (1000 μM) were applied via the same glass-polytetrafluoroethylen perfusion system, but from separate reservoirs. The contents of these reservoirs were mixed at a junction immediately before entering the superfusion chamber.

Drugs were applied either alone, in order to determine their direct agonistic effects, in combination with a sub-saturating glycine concentration (10 μM), in order to determine their co-activating effects, or together with a saturating (1000 μM) concentration of glycine in order to detect open channel block. A new cell was used for each drug and each protocol, at least three different experiments were performed for each setting. The amount of the diluent ethanol corresponding to the highest drug concentration used was 34 000 μM. We have previously shown that the ethanol itself has no effect at this concentration- neither on glycine receptor co-activation, nor on direct activation.

*1.1.4 Current recording and analysis*

For data acquisition and further analysis the inventors used the Axopatch 200B amplifier in combination with pClamp6 software (Axon Instruments, Union City, CA, USA). Currents were filtered at 2 kHz. Fitting procedures were performed using a nonlinear least-squares Marquardt-Levenberg algorithm. Details are provided in the appropriate figure legends or in the results section.

The maximum current response induced by a compound acting directly as an agonist was expressed as percentage of the maximum response to 1000 μM glycine in the absence of drug immediately following the respective test experiment. The co-activating effect was expressed as percentage of the current elicited by 10 μM glycine according to E (%) = 100 $[(I-I_0)/I_0]$, where $I_0$ is the current response to 10 μM glycine. Activated or co-activated currents were normalized to their own maximum response. For the non-halogenated compounds, the dose-response curves did not always reach a plateau response, because phenol derivatives in concentrations larger than 3000 μM lead to a decline in seal resistance and thus, did not yield reliable results. In these cases, the maximum response was the response at the highest concentration of the test compound for which a reliable response could be recorded. The dose-response-curves were fitted according to ($I_{norm} = [1^{+}(EC_{50}/[C])^{nH}]^{-1}$), where $I_{norm}$ is the current induced either directly by the respective concentration [C] of the agonist, or co-activated ($I-I_0$) by the agonist-glycine (10 μM) mixture, normalized to the maximum inward current or maximum co-activated current ($I_{max}-I_0$), $EC_{50}$ is the concentration required to evoke a response amounting to 50% of their own maximal response and $n_H$ is the Hill coefficient.

*1.1.5 Statistics*

Only results obtained with $\alpha_1$ß receptors were enrolled in the statistical tests. As a consequence of the higher glycine sensitivity in $\alpha_1$ homomeric receptors, a maximum co-activating response (with respect to the effect of 1000 μM glycine) might occasionally be observed at low drug concentrations leading to an underestimation of the $EC_{50}$ values derived from Hill-fits in $\alpha_1$ homomeric receptors. Statistical analysis was performed in order to reveal differences in the maximum effect on the one hand and in the concentrations required to achieve half-maximum effect ($EC_{50}$) on the other between halogenated and non-halogenated analogues, between compounds with one vs. two methyl groups, or between compounds with ortho- or meso- position of the methyl groups with respect to the phenolic hydroxyl group, respectively. Curve fitting and parameter estimation of the Hill curves were performed using the program "PROC NLMIXED" of SAS Release 8.02. In this model, the "experiment" is treated as the subject variable and the parameter values ($EC_{50}$ and $n_H$)

are treated as normally distributed random factors. The mean differences of these parameters between two substances were entered into the common model as fixed shift parameters $\Delta EC_{50}$ and $\Delta n_H$, activated for all data of the 2nd data set. The corresponding (asymptotic) t-value was used to test the null hypothesis of no parameter difference against the two-sided alternative. The null hypothesis was rejected at $p<0.05$. All data are depicted as means ± SD.

A two sample t-test was applied to analyze significance of differences in the maximum potentiating effect between halogenated and non-halogenated, mono- and bimethylated or ortho- vs. meso-methylated structural analogues.

## 1.2 Results

A total of 94 cells were included in the study. Expression of rat $\alpha_1$ homomeric and $\alpha_1\beta$ mRNA in HEK 293 cells generated glycine receptors that showed glycine-activated inward current with amplitudes of -1.0 ± 0.5 nA in $\alpha_1$ and 1.3 ± 0.9 nA in $\alpha_1\beta$ receptors following saturating (1000 µM) concentrations of the natural agonist. Successful co-expression of the ß subunit was verified with picrotoxin 1000 µM co-applied with 1000 µM glycine after each experiment. In this experimental setting, picrotoxin 1000 µM blocked $\alpha_1$ homomeric receptors by 55 ± 0.05 % while $\alpha_1\beta$ receptors were hardly affected by picrotoxin (19 ± 0.05 % block). When α and ß cDNAs were used at a 1 : 10 ratio for co-transfection, successful co-expression of the ß-subunit verified with picrotoxin was 100 %. The current transient showed a fast increase, followed by a monophasic decay. The time constant of desensitization was 958 ± 250 ms in $\alpha_1$ homomeric and 1026 ± 212 ms in $\alpha_1\beta$ receptors. The respective steady-state current that did not desensitize in the presence of 1000 µM glycine was at 86 ± 6 % and 84 ± 8 % of the peak current amplitude.

When applied without glycine, only 2,6 dimethylphenol and 3,5 dimethylphenol directly activated receptor-mediated inward currents in a concentration-dependent manner. Currents reached 30 ± 12 % ($\alpha_1$, n = 3) and 38 ± 5 % ($\alpha_1$ß, n = 3) and 32 ± 6 % ($\alpha_1$, n = 3) and 33 ± 10 % ($\alpha_1$ß, n = 3) of the maximum glycine (1000 µM) response in the presence of high concentrations (3000 µM) of either 3,5 dimethylphenol or 2,6 dimethylphenol, respectively. The estimates for half-maximum concentrations ($EC_{50}$) were 1468 ± 208 and 1466 ± 83 µM for 3,5 dimethylphenol and 1410 ± 101 and 1549 ± 164 µM for 2,6 dimethylphenol in $\alpha_1$ and $\alpha_1$ß receptors, respectively.

As illustrated by the tracings in Fig.2, currents induced by both compounds did not desensitize during the 2 s application.

Dose-response curves for glycine at $\alpha_1$ and $\alpha_1$ß receptors are shown in Fig.3. The $EC_{50}$ for glycine was 12.8 ± 2.3 µM at $\alpha_1$ and 47.0 ± 14.0 µM at $\alpha_1$ß receptors, respectively. Glycine 10 µM evoked a current response of 21 ± 7 % (n = 34) in $\alpha_1\beta$ and 46 ± 5 % (n = 24) of the response to 1000 µM glycine in $\alpha_1$ receptors, this difference in glycine sensitivity was significant (p<0.001). Less than 10% of the current response to 10 µM glycine desensitized as long as glycine was present.

All phenol derivatives investigated potentiated the current response to glycine 10 µM in both $\alpha_1$ and $\alpha_1\beta$ receptors, Figs. 4 and 5 show representative current traces obtained with $\alpha_1\beta$ receptors, Fig. 6 shows current traces obtained with $\alpha_1$ homomeric receptors.

No significant differences between the compounds were detected with respect to the degree of maximum potentiation. Only the potentiating effect seen with 3 methylphenol was higher than with 2 methylphenol (p =0.04), which, however, might be a consequence of the lower response to glycine 10 µM in the experiments with 3 methylphenol with respect to the experiments with 2 methylphenol.

The halogenated compounds 3,5 dimethyl-4-chlorophenol and 3 methyl-4-chlorophenol achieved half-maximum potentiating effects at more than 20-fold lower concentrations compared with their non-halogenated analogues, this difference was statistically significant (p<0.0001). The estimates for the $EC_{50}$ values for the compounds with the methyl groups in the meso position (3 methylphenol and 3,5 dimethylphenol) in $\alpha_1\beta$ receptors were not significantly different from the $EC_{50}$ values for their ortho-methylated structural analogues (2 methylphenol and 2,6 dimethylphenol). The non-halogenated bimethylated compounds were not significantly more potent than their structural analogues with only one methyl group in $\alpha_1$ß receptors. The concentration-dependence of current potentiation in $\alpha_1\beta$ receptors derived from 5-6 experiments for each compound is depicted in Fig. 7.

The $EC_{50}$ values and Hill-coefficients (± SD) derived from fits of the Hill equation to the normalized response in $\alpha_1$ and $\alpha_1$ß receptors are depicted in Table 1.

**Table 1** $EC_{50}$ values and Hill coefficients (+s.d.) derived from fits of the Hill equation to the normalised coactivating response (with respect to the effect of the highest concentration tested) in $\alpha_1$ and $\alpha_1\beta$ receptors

| | *$\alpha_1$ homomer* | | *$\alpha_1\beta$ heteromer* | |
|---|---|---|---|---|
| | *$EC_{50}$* (µM) | $n_H$ | *$EC_{50}$* (µM) | $n_H$ |
| 3 methyl-4-chlorophenol | 8±5 | 1.1±0.4 | **4±1*** | 1.2±0.3 |
| 3 methylphenol | 59±19 | 0.9+0.3 | 222±45 | 1.0±0.1 |
| 3,5 dimethyl-4-chlorophenol | 13±4 | 1.4±2.9 | **11+2*** | 1.3±0.1 |

(continued)

| | $\alpha_1$ *homomer* | | $\alpha_1\beta$ *heteromer* | |
|---|---|---|---|---|
| | $EC_{50}$ ($\mu$M) | $n_H$ | $EC_{50}$ ($\mu$M) | $n_H$ |
| 3,5 dimethylphenol | 254±139 | 1.7±0.9 | 308±46 | 1.3±0.2 |
| 2 methylphenol | 70±29 | 0.7±0.1 | 448±89 | 1.2±0.2 |
| 2,6 dimethylphenol | 226±104 | 1.5±0.4 | 373±51 | 1.2±0.1 |

In Table 1: the halogenated compounds 3 methyl-4-chlorophenol and 3,5 dimethyl-4-chlorophenol were significantly more potent than their nonhalogenated structural analogues (bold and *, P<0.0001). No significant differences were detected between compounds with one vs two methyl groups or between ortho- vs meso-methylated compounds in $\alpha_1\beta$ receptors.

As a consequence of the higher glycine sensitivity in $\alpha_1$ homomeric receptors, a maximum co-activating response (with respect to the effect of 1000 $\mu$M glycine) might occasionally be observed at low drug concentrations leading to an underestimation of the $EC_{50}$ values derived from Hill-fits in $\alpha_1$ homomeric receptors- thus, the parameters given for the $\alpha_1$ homomeric receptors should not be used for potency determinations. However, as revealed by the current traces in Figs 4, 5 and 6 and by the values given in Table 1, all phenol derivatives co-activate currents via $\alpha_1$ homomeric receptors in a similar concentration range compared to $\alpha_1$ß receptors, thus, the expression of the ß-subunit is not required for the co-activating effects.

The halogenated compounds 3,5 dimethyl-4-chlorophenol and 3 methyl-4-chlorophenol in concentrations larger than 300 and 600 $\mu$M, respectively, produced a reduction in the peak current amplitude when co-applied with 1000 $\mu$M glycine along with a large response rebound when co-application was terminated. The current decay was accelerated during co-application of the respective compound and glycine (1000 $\mu$M). A total of 3 experiments was performed for each compound to substantiate this effect. Figure 8 shows representative current traces.

## 1.3 Discussion

These data shows that substituted phenol derivatives that carry a chloride in the para position to the phenolic hydroxyl group co-activate glycine receptors at low concentrations and thus, may offer a potential for therapy of spasticity, muscle relaxation, and pain relief. At much higher concentrations, only the bi-methylated and non-halogenated compounds directly activated the glycine receptor in the absence of the natural agonist. These results show that direct activation and co-activation of glycine receptors by phenol derivatives require distinct structural features. The presence of the ß subunit is neither required for positive modulation nor for direct activation of glycine receptors by phenol derivatives.

$GABA_A$ and glycine receptors are the main receptors for inhibitory neurotransmission in the mammalian central nervous system. $GABA_A$ is the most important neurotransmitter in the brain and glycine plays a major role in the spinal cord and lower brain stem. While $GABA_A$ receptors have been identified as a common target site for structurally diverse sedative-anaesthetic and anxiolytic drugs, clinically applicable compounds that specifically target glycine receptors have yet to be identified. Glycine receptors have been suggested as potential candidates for therapeutics that mediate antinociceptive and muscle relaxant effects.

All phenol derivatives investigated in this study were capable of positively modulating glycine receptor function to a certain extent. Apparently, one important structural feature that determines the potency of a phenol derivative to co-activate glycine receptors is halogenation in the para position to the phenolic hydroxyl group. Insertion of a second symmetrical methyl group did not further increase the potency of the single-methylated compound, and the position of the methyl group with respect to the phenolic hydroxyl group had no influence on the co-activating potency. At higher concentrations (>300 $\mu$M), the co-activating effect of the halogenated compounds was overridden by inhibitory effects revealed by a reduction in the peak current amplitude during co-application with 1000 $\mu$M glycine. The large response rebound when co-application was stopped simultaneously is consistent with the assumption of open channel block as underlying mechanism- a phenomenon that has previously been described for the modulation of glycine receptors by high concentrations of propofol as well as for the modulation of $GABA_A$ receptors by inhalational agents. However, further studies should target voltage-dependence of these effects in order to substantiate the hypothesis of open-channel block. Alternatively, the reduction in peak current amplitude along with the acceleration of the current decay during co-application with 1000 $\mu$M glycine might be explained by an allosteric mechanism of inhibition with high concentrations of halogenated phenol derivatives stabilizing the desensitized conformation of the receptor, analogous to a mechanism of block assumed for picrotoxin on ligand-gated chloride channels. None of the halogenated compounds directly activated the receptor in the absence of the natural agonist.

The structural features that determine the potency of a phenol derivative to activate or co-activate $Cl^-$ inward

currents via glycine receptors show similarities as well as differences with respect to the requirements that have previously been reported for activation of GABA-ergic receptors or sodium channel blocking effects.

Qualitatively, the structure-activity relationship for co-activation of glycine receptors by phenol derivatives shows parallels with the structure-activity relationship to block voltage-operated sodium channels. In both cases, potency is strongly increased by the chloride in para position to the phenolic hydroxyl. Quantitatively, the half-maximum concentrations for glycine receptor co-activation in this study were about 10-fold (3,5 dimethyl-4-chlorophenol) and 100-fold (3 methyl-4-chlorophenol) lower than the concentrations required for half-maximum blockade of sodium channels by these compounds. While insertion of a chloride in para position led to a parallel increase in the potency to co-activate glycine receptors and to block of voltage-operated sodium channels, this is not the case for the insertion of a 2$^{nd}$ methyl group. In contrast to the effect at glycine receptors, the potency for sodium channel blockade was increased when a second methyl group was attached in the meso position. As for the halogenated compound with one single methyl group in the meso position (3 methyl-4-chlorophenol), there is only little overlap in the concentration range where glycine receptor co-activation was observed in this study and the concentration range where sodium channel blockade was reported. For comparison, half-maximum effect at glycine receptors was achieved with 4 $\mu$M 3-methyl-4-chlorophenol, whereas half-maximum block of sodium channels in the resting state required 400 $\mu$M 3-methyl-4-chlorophenol. In the case of the non-halogenated phenol derivatives with two methyl groups, co-activation of glycine receptors was detected in the same concentration range as sodium channel blockade. In this study, a half-maximum co-activating effect was observed with 370 $\mu$M 2,6 dimethylphenol compared to 187 $\mu$M for half-maximum blockade of voltage-operated neuronal sodium channels at a membrane potential close to the physiological resting potential. At much higher concentrations (> 1000 $\mu$M), the bi-methylated compounds directly activated the glycine receptor in the absence of the natural agonist. If these results can be generalized, halogenated phenol derivatives should show glycine receptor co-activation at low concentrations, with increasing concentrations blockade of voltage-operated sodium channels and open channel block of glycine receptors. Non-halogenated bi-methylated phenol derivatives should both co-activate glycine receptors and block sodium channels at intermediate concentrations and should directly activate glycine receptors at high concentrations. Halogenated phenol derivatives with one single methyl group would be expected to be sodium channel blockers, while having facilitating effects at glycine receptors at concentrations where sodium channel blockade would still be small.

In contrast to the glycinergic effects seen in this study, GABA-ergic effects were hardly affected by substitution in the para position. GABA-ergic activity of phenolic compounds has been linked to the size and shape of alkyl groups in position 2 and 6 of the aromatic ring relative to the phenolic hydroxyl group, however, the effect of 3,5 di-alkyl-substitution has never been tested systematically. Direct activation of $GABA_A$ receptors was seen in the single methylated compound only when the methyl group was in the ortho position. Our study shows that, as far as direct activation of glycine receptors is concerned, at least two methyl groups are required for a detectable effect which is independent from their position with respect to the phenolic hydroxyl group.

In conclusion, these data indicate that phenol derivatives according to the invention, which are preferably selective for glycine receptors rather than voltage-operated sodium channels or $GABA_A$ receptors, show a desirable pattern of anti-nociceptive, muscle relaxant and local anaesthetic/anti-convulsant effects in the model use. A skilled person will recognise that this illustrates that compounds according to the invention will be useful in the control of a number of painful conditions as discussed herein.

## 1.4 Conclusions

1. Phenol derivatives constitute a family of neuroactive compounds. The aim of this study was to identify structural features that determine their modulatory effects at glycine receptors.

2. The inventors investigated the effects of four methylated phenol derivatives and two halogenated analogues on chloride inward currents via rat $\alpha_1$ and $\alpha_1\beta$ glycine receptors, heterologously expressed in HEK 293.

3. All compounds potentiated the effect of a sub-maximal glycine concentration in both $\alpha_1$ homomeric and $\alpha_1\beta$ glycine receptors. While the degree of maximum potentiation of the glycine 10 $\mu$M effect in $\alpha_1\beta$ receptors was not different between the compounds, the halogenated compounds achieved half-maximum potentiating effects in the low $\mu$M range- at more than 20-fold lower concentrations compared with their non-halogenated analogues ($p<0.0001$). The co-activating effect was overridden by inhibitory effects at concentration >300 $\mu$M in the halogenated compounds.

4. Only the bi-methylated compounds 2,6- and 3,5-dimethylphenol (at concentrations > 1000) $\mu$M directly activated both $\alpha$1 and $\alpha$1ß receptors up to 30 % of the maximum response evoked by 1000 $\mu$M glycine.

These results show that halogenation in the *para* position is a crucial structural feature for the potency of a phenolic compound to positively modulate glycine receptor function while direct activation is only seen with high concentrations of compounds that carry at least 2 methyl groups. The presence of the ß-subunit is not required for both effects.

## EXAMPLE 2

The inventors expanded the preliminary work summarised in Example 1 and endeavoured to further improve the efficacy of phenol derivatives as analgesics. They designed and tested a number of derivatives of propofol and found to their surprise that the nature of the substituents on the phenol ring at the 2, 3, 5 and 6 positions (i.e at $R_1$, $R_2$, $R_4$ and $R_5$ of General formula I) had a significant effect on the selectivity of the compounds for glycine receptors and on there efficacy for activating the glycine recptor. These experiments led them to realise the usefuleness of the compounds according to the first aspect of the invention. Data for preferred compounds according to the invention are presented below for illustrative purposes. Surprisingly these compounds have $EC_{50}$ values for co-activating glycine receptors that fall in the nanomolar range.

The Experimental protocols employed in Example 1 were repeated to investigate the efficacy of a number of further halogenated propofol derivatives on glycine receptor activation (and therefore pain control).

The inventors established that compounds according to general formula I with at least two of $R_1$, $R_2$, $R_4$ and $R_5$ comprising alkyl groups of between 2 and 13 carbon atoms were particularly effective for modulating glycine receptors. Such compounds are particularly preferred compounds for use according to the invention and exhibited analgesic properties that were even better than compounds according to general formula I with methyl groups at $R_1$, $R_2$, $R_4$ and/or $R_5$ (see above).

Illustrative data for preferred compounds 4-chloropropofol, 4-bromopropofol and 4-iodopropofol is set out below.

### 2.1: 4-chloropropofol

The experiments conducted in Example 1 were repeated using 4-chloropropofol (4-chloro- 2,6 di-isopropylphenol).

Figure 9 illustrates representative current traces showing co-activation of the current response to 10 μM glycine when 4-chloropropofol was co-applied with 10 μM glycine (3rd, 4th, 5th and 6th current trace from top). The first trace shows current elicited by a supramaximal glycine concentration (1000 μM) a-subunits of glycine receptors from the rat were coexpressed with human ß subunits in HEK293 cells. Small cells were studied in the whole-cell mode using an ultra-fast application device.

Figure 10 illustrates concentration-dependence of the coactivation of the current response to 10 μM glycine by 4-chloropropofol (mean / SD, n = 6). The results presented in Figure 10 were corroborated by repeating the tests carried out to provide the data shown in Figure 10 to provide the data shown in Figure 11. Thus, Figure 11 also illustrates concentration-dependence of the coactivation of the current response to 10 μM glycine by 4-chloropropofol (mean / SD, n = 5). Note from Figures 10 and 11 that the co-activating effect is observed in the concentration range between 1 and 100 nM. The co-activating effect is overridden by inhibitory effects at higher concentrations (≥1000 μM). The decline in the peak current amplitude at concentrations above 10 μM is probably due to open channel block at higher concentrations, a phenomenon that has been described for propofol. a-subunits of glycine receptors from the rat were coexpressed with human ß-subunitsin HEK293 cells. Small cells were studied in the whole-cell mode using an ultra-fast application device.

Figure 12 represents current traces elicited by 4-chloropropofol in the absence of the natural agonist glycine (2nd, 3rd and 4th trace from top) with respect to the current elicited by a supramaximal glycine concentration (1000 μM), top. a-subunits of glycine receptors from the rat were coexpressed with human ß-subunits in HEK293 cells. Small cells were studied in the whole-cell mode using an ultra-fast application device.

These data have also been confirmed in experiments using Oocytes instead of HEK293 cells as expression system.

Figure 13 shows the current directly activated (mean ± SD, n=4) by 4-chloropropofol normalized to its own maximum response (empty symbols) or the maximum response elicited by 1000 μM glycine (filled symbols). The solid line is a Hill fit to the data with the indicated parameters. Note that the direct activating effect is observed at 1000-fold higher concentrations than the co-activating effect.

### 2.2: 4-bromopropofol

The experiments conducted in Example 1 were repeated using 4-bromopropofol (4-bromo-2,6 di-isopropylphenol).

Figure 14 illustrates representative current traces showing co-activation of the current response to 10 μM glycine when 4-bromopropofol was co-applied with 10 μM glycine (3rd, 4th, 5th and 6th current trace from top). The first

trace shows current elicited by a supramaximal glycine concentration (1000 μM) a-subunits of glycine receptors from the rat were coexpressed with human ß subunits in HEK293 cells. Small cells were studied in the whole-cell mode using an ultra-fast application device.

Figure 15 illustrates concentration-dependence of the coactivation of the current response to 10 μM glycine by 4-bromopropofol (mean / SD, n = 5). Note from Figure 15 that the co-activating effect is observed in the concentration range between 1 and 100 nM. The co-activating effect is overridden by inhibitory effects at higher concentrations (≥1000 μM).

Figure 16 represents current traces elicited by 4-bromopropofol in the absence of the natural agonist glycine (2nd, 3rd and 4th trace from top) with respect to the current elicited by a supramaximal glycine concentration (1000 μM), top. a-subunits of glycine receptors from the rat were coexpressed with human ß-subunits in HEK293 cells. Small cells were studied in the whole-cell mode using an ultra-fast application device.

Figure 17 shows the current directly activated (mean ± SD, n=4) by 4-bromopropofol normalized to its own maximum response (empty symbols) or the maximum response elicited by 1000 μM glycine (filled symbols). The solid line is a Hill fit to the data with the indicated parameters. Note that the direct activating effect is observed at 1000-fold higher concentrations than the co-activating effect.

**2.3: 4-iodopropofol**

The experiments conducted in Example 1 were repeated using 4-iodopropofol (4-iodo-2,6 di-isopropylphenol).

Figure 18 illustrates representative current traces showing co-activation of the current response to 10 μM glycine when 4-iodopropofol was co-applied with 10 μM glycine (3rd, 4th, 5th and 6th current trace from top). The first trace shows current elicited by a supramaximal glycine concentration (1000 μM) a-subunits of glycine receptors from the rat were coexpressed with human ß subunits in HEK293 cells. Small cells were studied in the whole-cell mode using an ultra-fast application device.

Figure 19 illustrates concentration-dependence of the coactivation of the current response to 10 μM glycine by 4-iodopropofol (mean / SD, n = 4). Note from Figure 19 that the co-activating effect is observed in the concentration range between 1 and 100 nM. The co-activating effect is overridden by inhibitory effects at higher concentrations (≥1000 μM).

Figure 20 represents current traces elicited by 4-iodopropofol in the absence of the natural agonist glycine (2nd, 3rd and 4th trace from top) with respect to the current elicited by a supramaximal glycine concentration (1000 μM), top. a-subunits of glycine receptors from the rat were coexpressed with human ß-subunits in HEK293 cells. Small cells were studied in the whole-cell mode using an ultra-fast application device.

Figure 21 shows the current directly activated (mean ± SD, n=4) by 4-iodopropofol normalized to its own maximum response (empty symbols) or the maximum response elicited by 1000 μM glycine (filled symbols). The solid line is a Hill fit to the data with the indicated parameters. Note that the direct activating effect is observed at 1000-fold higher concentrations than the co-activating effect.

Figures 12, 13, 16, 17, 20 and 21 provide experimental data for the effect of the 4-chloro-, 4-bromo- and 4-iodo- derivatives of 2,6-di-isopropylphenol when provided in the absence of the natural transmitter glycine. The three compounds exhibited $EC_{50}$ values in the single-digit micromolar range, i.e. three orders of magnitude higher than in the presence of glycine (see Figures 11, 15 and 19). While the inventors do not wish to be bound by any specific theory, the results of these tests suggest that the three 4-halo-propofol derivatives do not exert a direct effect, but rather, that they modulate the effect of the natural transmitter on gating of glycine receptors.

**2.4 Further Experimental data**

Further experiments were conducted with 4-chloropropofol that confirmed the efficacy of compounds according to the invention. These included:

(a) Preliminary experiments 100nM 4-chloropropofol greatly enhanced the glycine (EC10)-evoked current mediated by recombinant glycine alpha1 receptors expressed in Xenopus oocytes. These data support the data about the effects of 4-chloropropofol on recombinant receptors expressed in human cell lines (HEK 293) but utilising a completely different expression system.

(b) In a further series of preliminary experiments using a rat *in vitro* spinal cord preparation 100nM - 1μM 4-chloropropofol produced a prolongation of the sIPSCs (mediated by synaptic glycine receptors) and an increase in a tonic conductance (mediated by extrasynaptic glycine receptors).Importantly these data suggest that the potent effects of 4-chloropropofol reported for recombinant receptors expressed in human cell lines as well as in Xenopus oocytes, are reproduced for native glycine receptors in a neuronal setting.

**EXAMPLE 3**

3.1 Introduction

The rhythmic swimming behaviour of *Xenopus laevis* frog embryos (Figure 22A) is recognised as a powerful model system for exploring spinal neural networks controlling locomotion. The intensity, frequency and duration of swimming episodes are regulated by two inhibitory pathways: a descending brainstem GABA pathway that turns off swimming and a spinal glycinergic pathway that controls the cycle periods attained during swimming. Potentiation of the GABA pathway reduces the duration of swimming episodes while potentiation of the glycinergic pathway (e.g. by noradrenaline or nitric oxide) increased cycle periods and hence slows swimming frequency.

The inventors realised that this model could be exploited to assess the relative efficacy of compounds to modulate either the GABA receptor or glycine receptors. Accordingly they were able to exploit the assay described below as a method of screening for compounds that are effective for modulating pain according to the invention.

The general anaesthetics etomidate and propofol were shown to exert an inhibitory effect upon swimming activity in *Xenopus* embryos by potentiating GABAergic synaptic pathways within the CNS. Propofol was also shown at higher concentrations (40$\mu$M) to mediate effects via actions at the postsynaptic glycine receptor. This demonstrated that propofol is a potent allosteric regulator of the $GABA_A$ in this system but also has actions at the glycine receptor.

In the present study *Xenopus* embryos were used to test the action of 4-chloropropofol (a compound according to the first asepect of the invention) upon fictive swimming in immobilized embryos. Initially 4-chloropropofol was applied in the absence of receptor antagonists to determine its anaesthetic properties compared to propofol and then the interactions of propofol with GABA and glycine receptors were assessed using bicuculline and strychnine, respectively.

**3.2 Methods**

Stage 37/8 embryos (Figure 22Ai) were immobilized in $\alpha$-bungarotoxin, secured on a Sylgard block mounted in a recording chamber and recirculated with frog ringer. The flank skin on the left and right sides of the trunk was removed to expose the inter-myotomal clefts wherein lie the ventral roots and then three glass suction electrodes were positioned at rostral and caudal levels on the left side and rostrally on the right side(Figure 22Aiii) to record "fictive" swimming (Figure 22B). Swimming activity was evoked by a brief 1 msec current pulse applied to the tail skin via a fourth glass suction electrode. Drugs were applied directly to the bath. Data was digitized using a CED 1401 interface, displayed using spike 2 software and analysed using Dataview software (Courtesy of W.J., Heitler, University of St. Andrews).

**3. 3 Results**

**3.3.1: 4-chloropropofol modulates fictive swimming**

Figure 23 shows the effect of 10$\mu$M 4-chloropropofol on fictive swimming. 4-chloropropofol significantly increased cycle periods during swimming (Figure 23Ai cf Aii; excerpts from end of each episode), an effect that persisted throughout each episode (Figure 22B) and that was partially but significantly reversed by a return to control saline. In data pooled from 5 experiments, cycle periods increase by approximately 20% on average. Rostro-caudal and left-right delay also increased by 8% and 19% respectively (not illustrated), but there was no significant change in episode duration (Figure 24B). The saline wash reversed these effects on cycle period by 25-30%, rostro-caudal delay by 12% and left-right delay by 22%. Since the cycle periods attained during swimming are regulated partly by the strength of reciprocal glycinergic inhibition these results indicate an ability of 10$\mu$M 4-chloropropofol to potentiate glycinergic neurotransmission and therefore demonstrates that a compound according to the invention had good activity at glycine receptors. Furthermore, the lack of significant effect on episode duration suggests that 4-chloropropofol exerts no or only a weak influence upon GABAergic neurotransmission. This demonstrates that a compound according to the first aspect of the invention has good selectivity for glycine receptors, over GABA receptors in an *in vivo* model and is therefore useful for pain management. The selectivity of 4-chloropropofol is in contrast to the more potent effects of the anaesthetic, propofol on GABA in this system. However, in order to better establish the action of the anaesthetic, subsequent experiments utilised antagonists for firstly the $GABA_A$ receptor (bicuculline methiodide) and then glycine receptor (strychnine).

**3.3.2: 4-chloropropofol effects are resistant to $GABA_A$ receptor blockade**

Figure 25 shows excerpts of ventral root activity after exposure to 40$\mu$M bicuculline methiodide to block $GABA_A$ receptors (A), after the addition of 10$\mu$M 4-chloropropofol in the presence of bicuculline (B). The effects of 4-chloropropofol on cycle periods clearly persisted after $GABA_A$ receptor blockade. In this experiment, 4-chloropropofol increased episode duration (A) by 2 seconds on average and cycle periods by 8.2 ms at the start of the episode (Ai cf. Bi) and by 20.3ms

at the end of the episode (Aii cf. Bii). The bicuculline wash (not illustrated) reduced episode duration by 2 seconds and cycle period at the start of the episode by 2ms but at the end of the episode cycle periods increased by a further 6.5 ms. Bicuculline produces a short episode of fast swimming with an average cycle period of 50.3ms, which is increased to 61.3 ms 20 minutes after the addition of 4-chloropropofol, an effect that was reversed by the bicuculline methiodide wash to 54.5ms.

These results demonstrate that 10μM 4-chloropropofol mediates the majority of its actions through the potentiation of a different inhibitory receptor to the $GABA_A$ receptor. In order to verify that the glycine receptor plays an important role, 1μM Strychnine was next applied.

### 3.3.3 4-chloropropofol potentiates glycinergic pathways

The lengthening of cycle periods by 4-chloropropofol in the presence of bicuculline suggests an action of the compound upon glycinergic transmission, for which the following experiments provide strong supporting evidence. Figure 27 shows excerpts of ventral root activity 20 minutes after applying 1μM strychnine (Ai) and 40 minutes after applying 10μM 4-chloropropofol (Aii). In the presence of the compound (Figure 27Aii), episode duration decreased by 17 seconds (A), whereas cycle periods at the start of the episode increased by an average of only 0.5ms (Bi) and at the end of the episode decreased by an average of 3.4 ms (Bii). The strychnine wash (not illustrated) increased episode duration by 2 seconds (A) and decreased cycle periods at the start (Bi) and end of the episode (Bii) by 3 and 5.4 ms respectively. Figure 27B illustrates the lack of effect of 4-chloropropofol on cycle periods throughout an episode of swimming. Pooled data (n=3) were used to examine the effect on the four parameters of fictive swimming when the compound is applied in the presence of 1μM strychnine. 4-chloropropofol decreased cycle periods, but not significantly, by less than 2%, which decreased a further 3% by the strychnine wash. There was little or no effect mediated by 4-chloropropofol on episode duration (B) or left-right delay (D).

### 3.3.4 Summary

At a concentration of 10μM 4-chloropropofol potentiates inhibition mediated by glycinergic pathways, supported by its effects on the parameters of fictive swimming before and after the application of antagonists Bicuculline methiodide (40μM) and Strychnine (1μM). When applied alone, the compound inhibits fictive swimming through potentiating inhibitory pathways that bring about an increase in cycle periods, rostro-caudal and left-right delay with no change on episode duration. After blocking $GABA_A$ receptors with 40μM Bicuculline methiodide, 4-chloropropofol exerts a similar effect upon fictive swimming continuing to increase cycle periods and left-right delay by approximately the same percentage an effect that is reversed by the wash. Again there is little change in episode duration, but for these experiments there is a clear decrease in rostro-caudal delay. In order to determine the ability of 4-chloropropofol to potentiate glycinergic neurotransmission, 1μM strychnine was used to block glycine receptors. There was a fractional decrease in cycle periods, no change in episode duration or left-right delay by the compound or the wash and only a small increase in rostro-caudal delay. These results provide supportive evidence for the ability of 4-chloropropofol to inhibit fictive swimming through potentiation of glycinergic pathways and to a lesser extent through the enhancement of GABAergic neurotransmission.

## 3.4 Discussion

Fictive swimming in stage 37/8 *Xenopus laevis* embryos is co-ordinated by a central pattern generator (CPG) primarily located within the spinal cord. This network comprises descending and commissural interneurons, and motor neurons. These neurons on opposite sides of the spinal cord fire in strict alternation thereby producing alternate muscle contractions of antagonistic sides. Excitation for swimming is produced by glutamatergic descending interneurons. The left-right alternation is brought about by the activity of glycinergic commissural interneurons mediate mid-cycle reciprocal inhibition of each CPG half centre. Fictive swimming episodes may terminate spontaneously or as a result of GABAergic mid-hindbrain reticulospinal neurons activated by pressure to the rostral cement gland which release GABA in the spinal cord to activate $GABA_A$ receptors. The purpose of this study was to determine the effect of 4-chloropropofol, upon inhibitory neurotransmission within the CNS of stage 37/8 *Xenopus laevis* embryos.

### 3.4.1: 4-chloropropofol inhibits fictive swimming

4-chloropropofol (a compound according to the invention) is a derivative of the anaesthetic propofol and was expected to have a similar inhibitory effect upon the CNS of the *Xenopus* embryo, potentiating the $GABA_A$ and glycine receptors and thereby inhibiting fictive swimming. However, as discussed in this example, 4-chloropropofol was found to have distinct effects in that it exhibited selectivity for glycine receptors (aassayed as an inhibition of fictive swimming) and thereby demonstrates a utility of 4-chloropropofol as an analgesic.

The first set of experiments investigate the effects of 10μM 4-chloropropofol; pooled data (n=5) revealed an increase in cycle period, rostro-caudal and left-right delay with no change in episode duration. After a wash in saline measurements of these parameters returned to control levels. The main effect was a decrease in swim frequency.

Propofol potentiates GABAergic neurotransmission to a greater extent than glycinergic and from this evidence a skilled person may have inferred that 4-chloropropofol would also significantly potentiate $GABA_A$ transmission and thereby act as an anaestheic. However subsequent experiments explored the effects of 10μM P-Cl-propofol in the presence of 40μM Bicuculline methiodide and illustrated that this surprisingly was not the case. In this experiment 4-chloropropofol gave similar results to the experiments described above comparing control saline to the compound, where cycle periods increased by a similar amount but episode duration did not alter. The effects on cycle period were reversible after returning to bicuculline alone. From this it may be concluded that 4-chloropropofol does not exert its effects on the fictive swimming frequency through allosteric binding interactions with the $GABA_A$ receptor. Instead 4-chloropropofol binds to allosteric sites on the glycine receptor potentiating these inhibitory pathways and reducing fictive swimming frequency as a result.

The last set of experiments tested the hypothesis that 4-chloropropofol, and other compounds according to the invention, potentiates glycinergic neurotransmission by pre-blocking glycine receptors with 1μM strychnine and then applying the compound. The results (n=3) revealed that the effects of 4-chloropropofol on cycle periods are occluded by strychnine; cycle periods decreased by less than 2% after addition of the 4-chloropropofol and decreased a further 1% with the strychnine wash. There was no change in episode duration or left-right delay when 4-chloropropofol was present. These results demonstrate that 10μM 4-chloropropofol increases cycle period, rostro-caudal delay and left-right delay by enhancing glycinergic inhibition.

### 3.5 Conclusions

4-chloropropofol reduces the frequency of fictive swimming in *Xenopus* embryos, an effect which persists throughout each episode. However, the duration of each episode was not significantly affected. These effects are explained by a potentiation of glycinergic transmission and demonstrates in an *in vivo* model that compounds according to the invention are selective for glycine receptors and will therefore be useful as analgesics rather than the GABA selectivity of propofol which is useful as an anaethetic.

Changes in the strength of glycinergic inhibition are known to exert a potent influence of swimming frequency. For example, a reduction in the inhibition (e.g. by applying strychnine) increases swimming frequency while potentiating glycinergic synapses (e.g. with noradrenaline or nitric oxide) reduces swim frequency. The effects of 4-chloropropofol cannot be accounted for by an influence on GABA neurotransmission since i) it does not significantly affect episode duration and ii) the effects persist in the presence of bicuculline at concentrations sufficient to block $GABA_A$ receptors. In contrast, applications of strychnine occluded the effects of 4-chloropropofol on swimming.

These effects of 4-chloropropofol indicate a different primary site of action to propofol, which inhibited swimming via actions at the $GABA_A$ receptor at low concentration (1μM), but only affected swimming via potentiation of the glycine receptor at higher concentrations (40μM). 4-chloropropofol appears to have a negligible effect at the $GABA_A$ receptor at 10μM but exerts a powerful influence on glycine receptors at this concentration. This clearly demonstrates the surprising receptor selectivity of compounds according to the invention compared to propofol.

### EXAMPLE 4

The inventors developed an *in vivo* pain model for further evaluation of the efficacy of the compounds according to the invention

### 4.1 Methods

Male Sprague-Dawley rats, approx 250g, were used to test whether not the compounds had analgesic properties.

**4.1.1 Pilot study:** Test animals will receive a Chronic Constriction Injury (CCI) with test compound or vehicle administered via intrathecal canulae (IC) according to standard protocols.

Tests will be carried out on two experimental groups (n+6/group):

CCI + Intrathecal Canulae (IC) + vehicle

CCI + Intrathecal Canulae (IC) + Highest of three doses

Animals are monitored for signs of toxicity with the vehicle and the compound. Animal behaviour is also assessed.

### 4.1.2 Main experiment:

The Bennett model, which is known to the art, of neuropathic pain (loose ligation of one sciatic nerve) is employed.
Outcome measures will be thermal, von Frey or paw pressure (as described in Randell & Selitto (1957) Arch Int Pharmacodyn 4: p409-421).
5 groups (N=8 each group) will be tested comprising: Vehicle; Compound dose 1; Compound dose 2; Compound dose 3; Positive control (gabapentin 100 mg/kg/day)
Obtain values at baseline, post injury (day 7). Then commence continuous intrathecal drug treatment via miniosmotic pumps. Three further behavioural tests at days 8, 10, and 14.

### 4.2 Results

The inventors expect compounds according to the invention to have analgesic properties according to the Randell-Selitto test criteria.

## Claims

1. Use of a compound selected from the group consisting of: 4-bromopropofol, 4-chloropropofol and 4-iodopropofol, and salts thereof; in the manufacture of a medicament for the treatment of pain.

2. The use according to claim 1 wherein the treatment of pain is treatment of existing pain.

3. The use according to claim 1 wherein the treatment of pain is prophylactic treatment of pain.

4. The use according to any preceding claim, wherein the pain is chronic pain.

## Patentansprüche

1. Verwendung einer Verbindung ausgewählt von der Gruppe bestehend aus:

   4-Bromopropofol, 4-Chlorpropofol und 4-Jodpropofol und Salzen davon; in der Herstellung eines Medikaments zur Schmerzbehandlung.

2. Verwendung nach Anspruch 1, wobei die Schmerzbehandlung eine Behandlung von vorhandenem Schmerz ist.

3. Verwendung nach Anspruch 1, wobei die Schmerzbehandlung eine prophylaktische Behandlung von Schmerz ist.

4. Verwendung nach einem vorherigen Anspruch, wobei der Schmerz ein chronischer Schmerz ist.

## Revendications

1. Utilisation d’un composé sélectionné dans le groupe constitué par : le 4-bromopropofol, le 4-chloropropofol et le 4-iodopropofol et leurs sels ; lors de la fabrication d’un médicament destiné au traitement de la douleur.

2. Utilisation selon la revendication 1, dans laquelle le traitement de la douleur est un traitement d’une douleur existante.

3. Utilisation selon la revendication 1, dans laquelle le traitement de la douleur est un traitement prophylactique d’une douleur.

4. Utilisation selon l'une quelconque des revendications précédentes, dans laquelle la douleur est une douleur chronique.

**FIG.1**

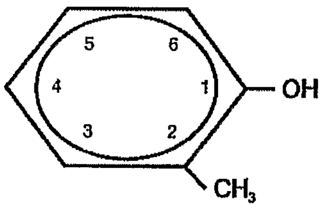

2 methylphenol

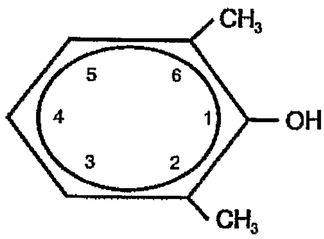

2,6 dimethylphenol

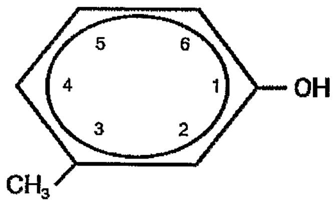

3 methylphenol

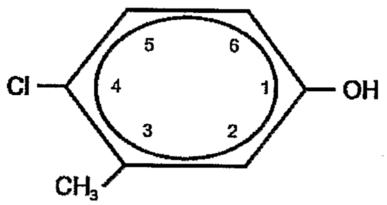

3 methyl-4-chlorophenol

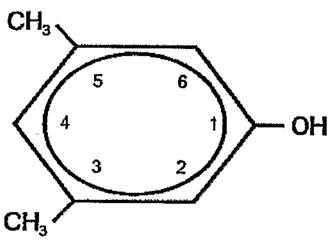

3,5 dimethylphenol

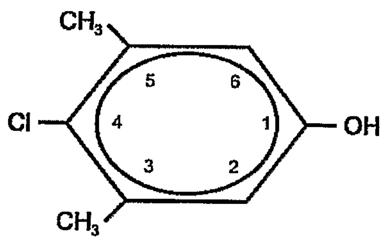

3,5 dimethyl-4-chlorophenol

FIG.2

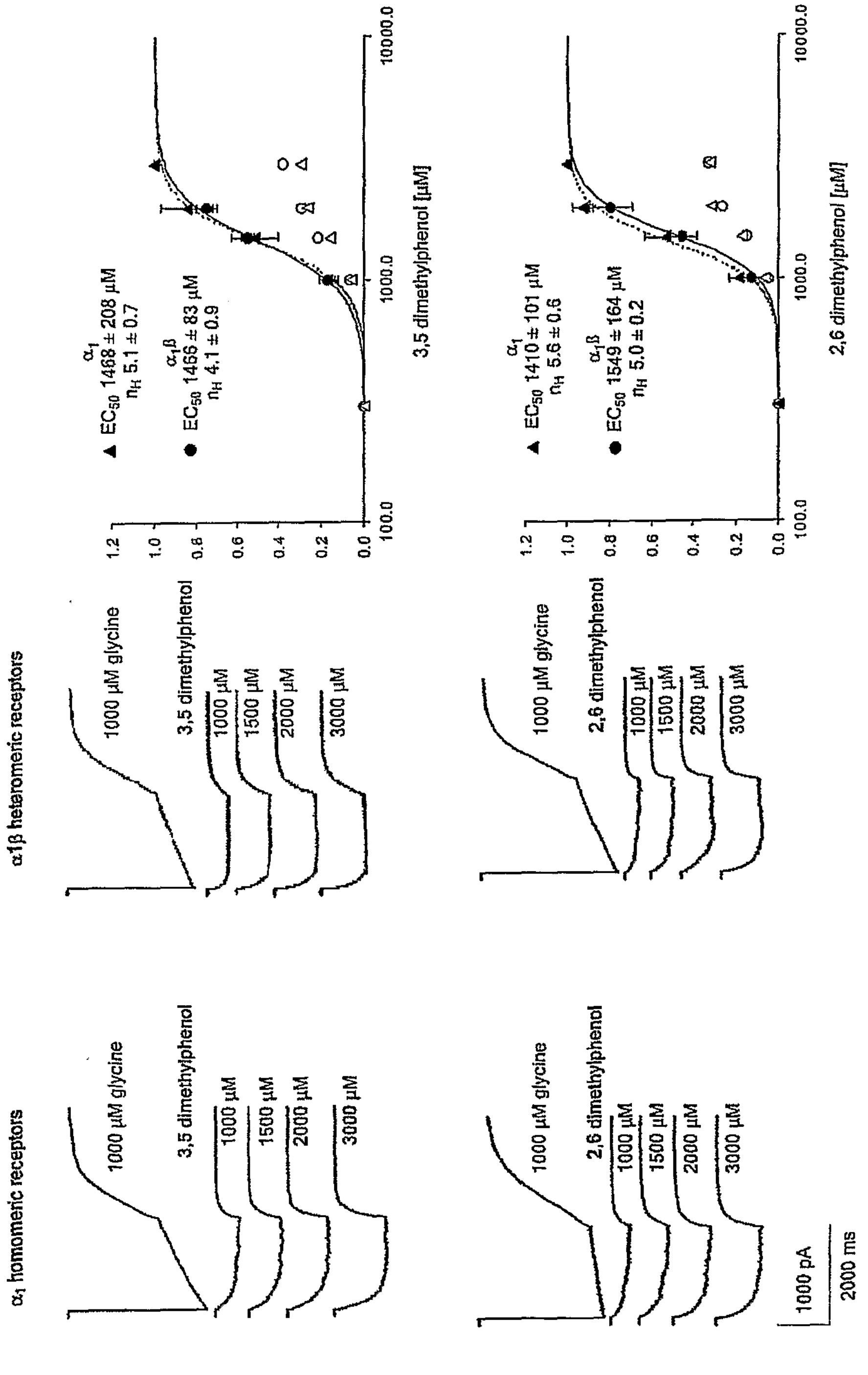

α1 homomeric receptors
1000 µM glycine
3,5 dimethylphenol
1000 µM
1500 µM
2000 µM
3000 µM
2,6 dimethylphenol
1000 pA
2000 ms
α1β heteromeric receptors
α1 EC50 1468 ± 208 µM nH 5.1 ± 0.7
α1β EC50 1466 ± 83 µM nH 4.1 ± 0.9
3,5 dimethylphenol [µM]
α1 EC50 1410 ± 101 µM nH 5.6 ± 0.6
α1β EC50 1549 ± 164 µM nH 5.0 ± 0.2
2,6 dimethylphenol [µM]

**FIG. 3**

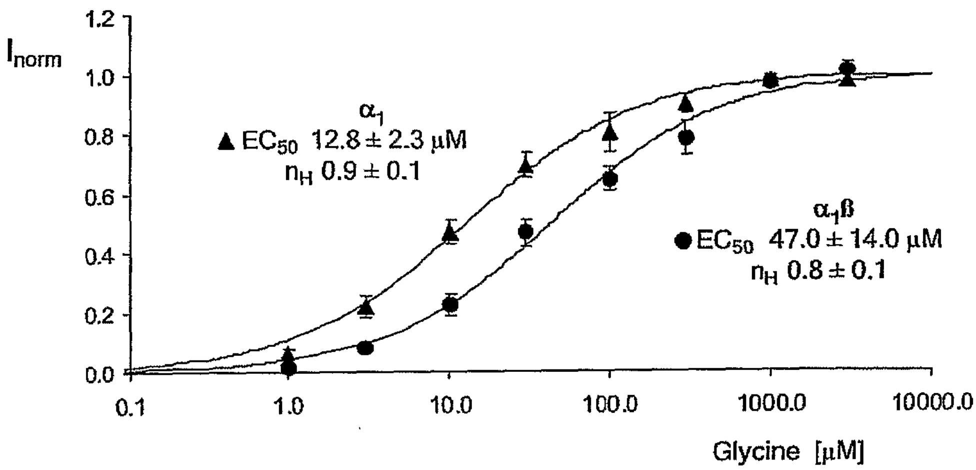

Inorm
1.2
1.0
0.8
0.6
0.4
0.2
0.0
α1
▲ EC50 12.8 ± 2.3 μM
nH 0.9 ± 0.1
α1β
● EC50 47.0 ± 14.0 μM
nH 0.8 ± 0.1
0.1
1.0
10.0
100.0
1000.0
10000.0
Glycine [μM]

FIG. 4

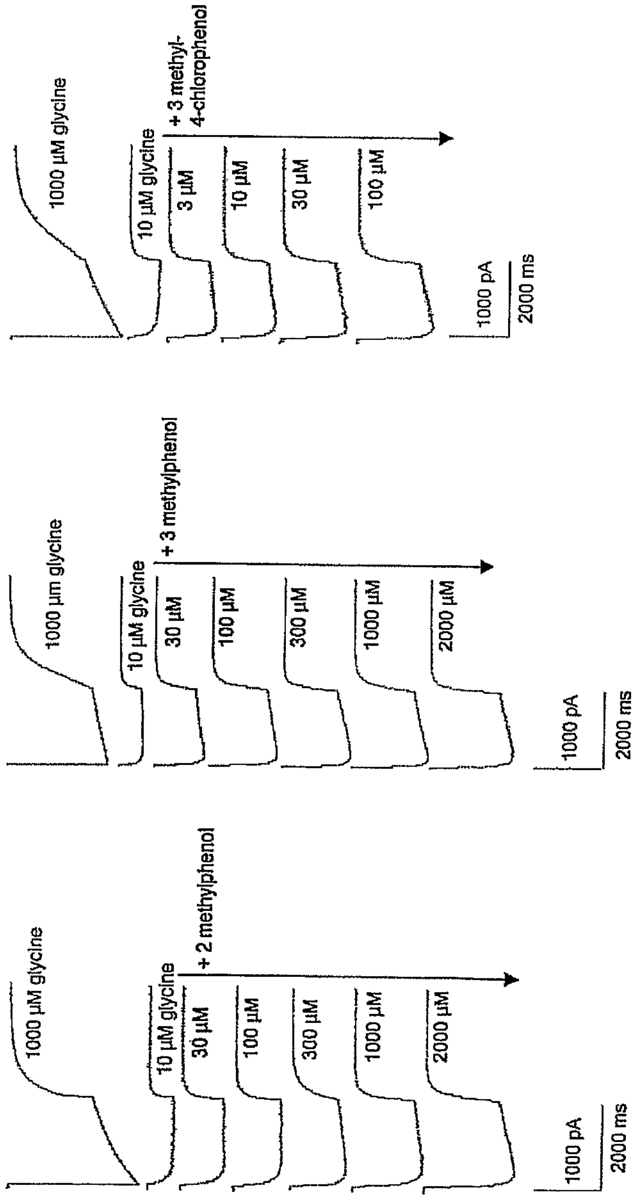
1000 µM glycine
10 µM glycine
+ 3 methyl-
4-chlorophenol
3 µM
10 µM
30 µM
100 µM
1000 pA
2000 ms
1000 µm glycine
10 µM glycine
+ 3 methylphenol
30 µM
100 µM
300 µM
1000 µM
2000 µM
1000 pA
2000 ms
1000 µM glycine
10 µM glycine
+ 2 methylphenol
30 µM
100 µM
300 µM
1000 µM
2000 µM
1000 pA
2000 ms

FIG. 5

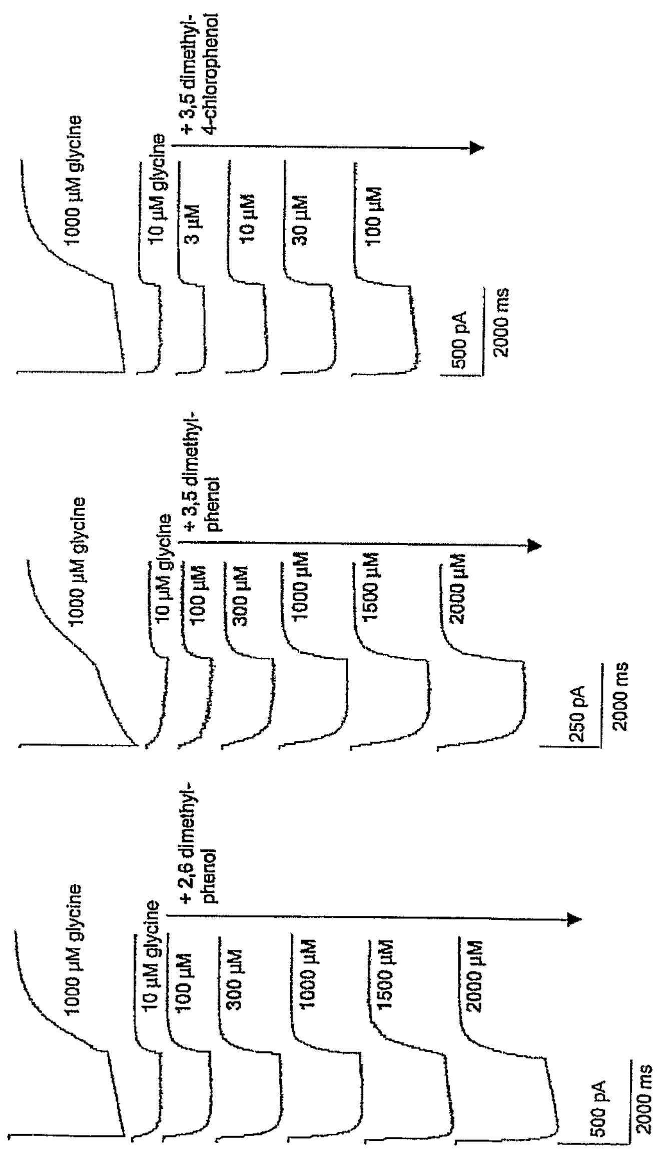
1000 µM glycine
10 µM glycine
+ 3,5 dimethyl-
4-chlorophenol
3 µM
10 µM
30 µM
100 µM
500 pA
2000 ms
1000 µM glycine
10 µM glycine
+ 3,5 dimethyl-
phenol
100 µM
300 µM
1000 µM
1500 µM
2000 µM
250 pA
2000 ms
1000 µM glycine
10 µM glycine
+ 2,6 dimethyl-
phenol
100 µM
300 µM
1000 µM
1500 µM
2000 µM
500 pA
2000 ms

FIG. 6

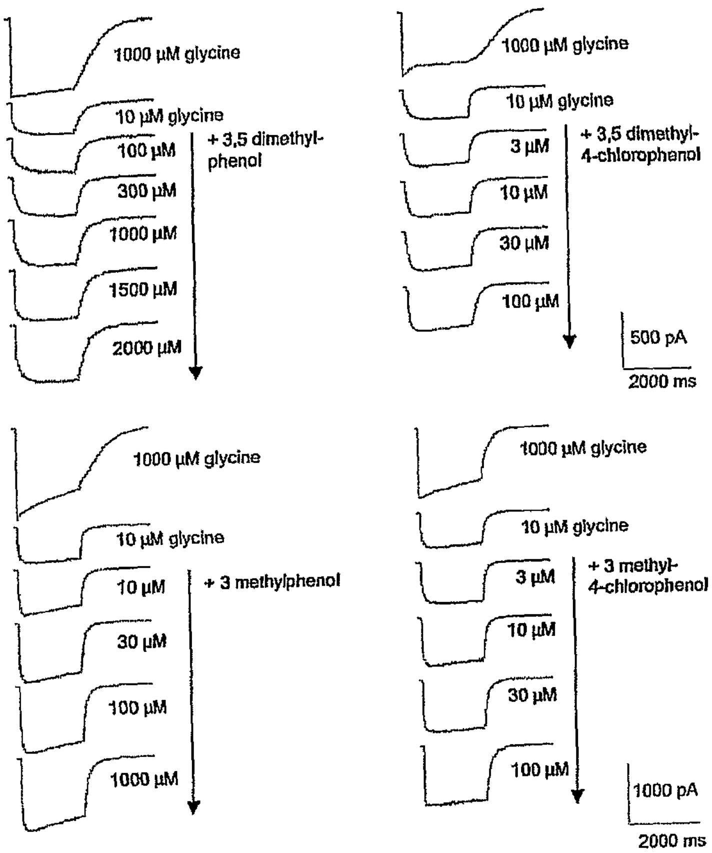
1000 µM glycine
10 µM glycine
+ 3,5 dimethyl-phenol
100 µM
300 µM
1000 µM
1500 µM
2000 µM
1000 µM glycine
10 µM glycine
+ 3,5 dimethyl-4-chlorophenol
3 µM
10 µM
30 µM
100 µM
500 pA
2000 ms
1000 µM glycine
10 µM glycine
+ 3 methylphenol
10 µM
30 µM
100 µM
1000 µM
1000 µM glycine
10 µM glycine
+ 3 methyl-4-chlorophenol
3 µM
10 µM
30 µM
100 µM
1000 pA
2000 ms

FIG. 7

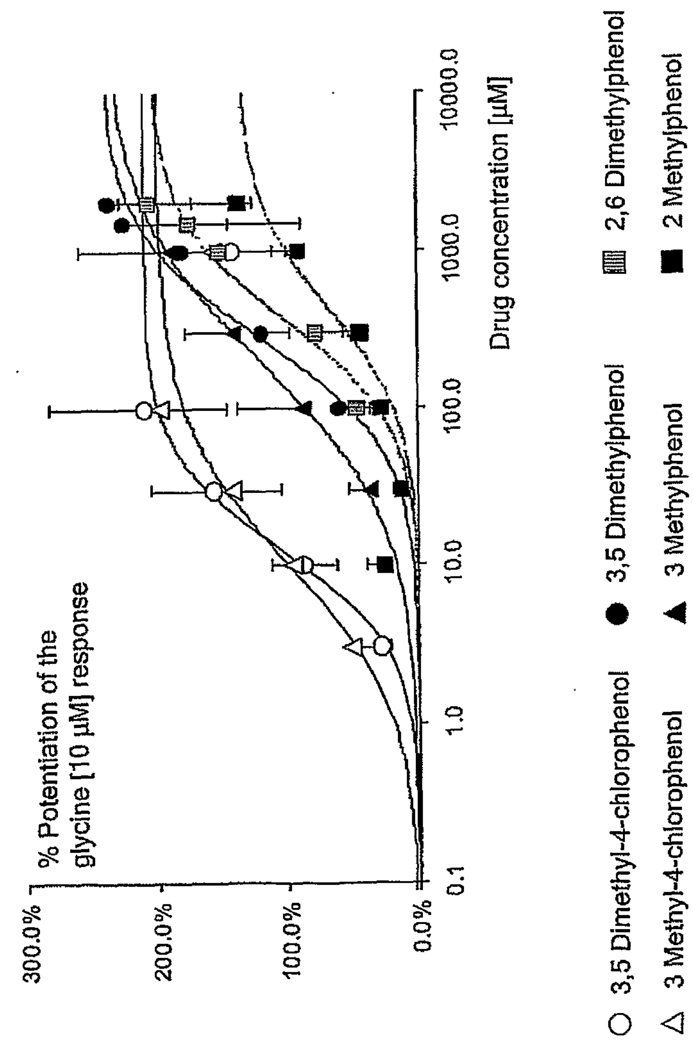
% Potentiation of the
glycine [10 µM] response
300.0%
200.0%
100.0%
0.0%
0.1
1.0
10.0
100.0
1000.0
10000.0
Drug concentration [µM]
3,5 Dimethyl-4-chlorophenol
3 Methyl-4-chlorophenol
3,5 Dimethylphenol
3 Methylphenol
2,6 Dimethylphenol
2 Methylphenol

**FIG. 8**

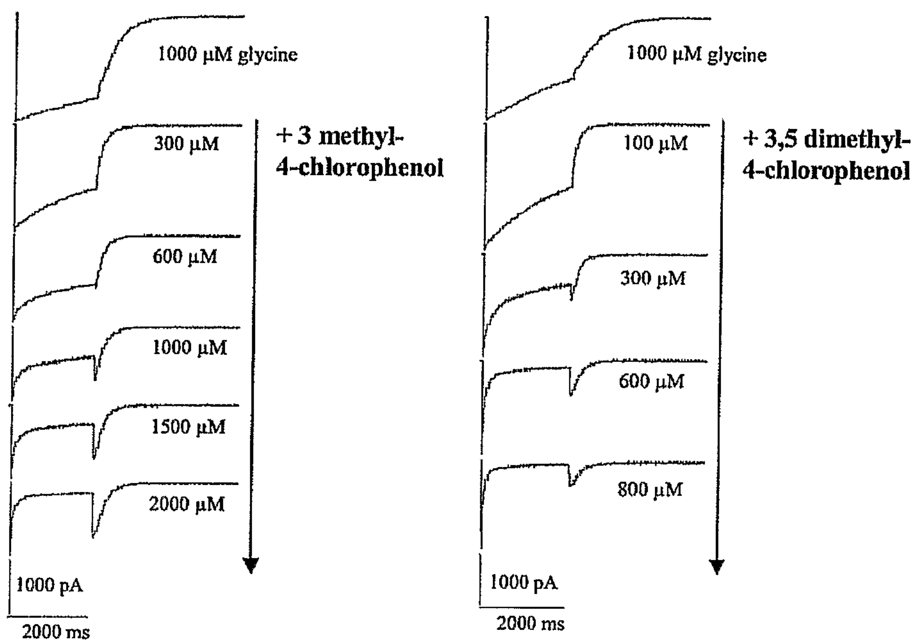
1000 μM glycine
300 μM
600 μM
1000 μM
1500 μM
2000 μM
+ 3 methyl-4-chlorophenol
1000 pA
2000 ms
1000 μM glycine
100 μM
300 μM
600 μM
800 μM
+ 3,5 dimethyl-4-chlorophenol
1000 pA
2000 ms

FIG. 9

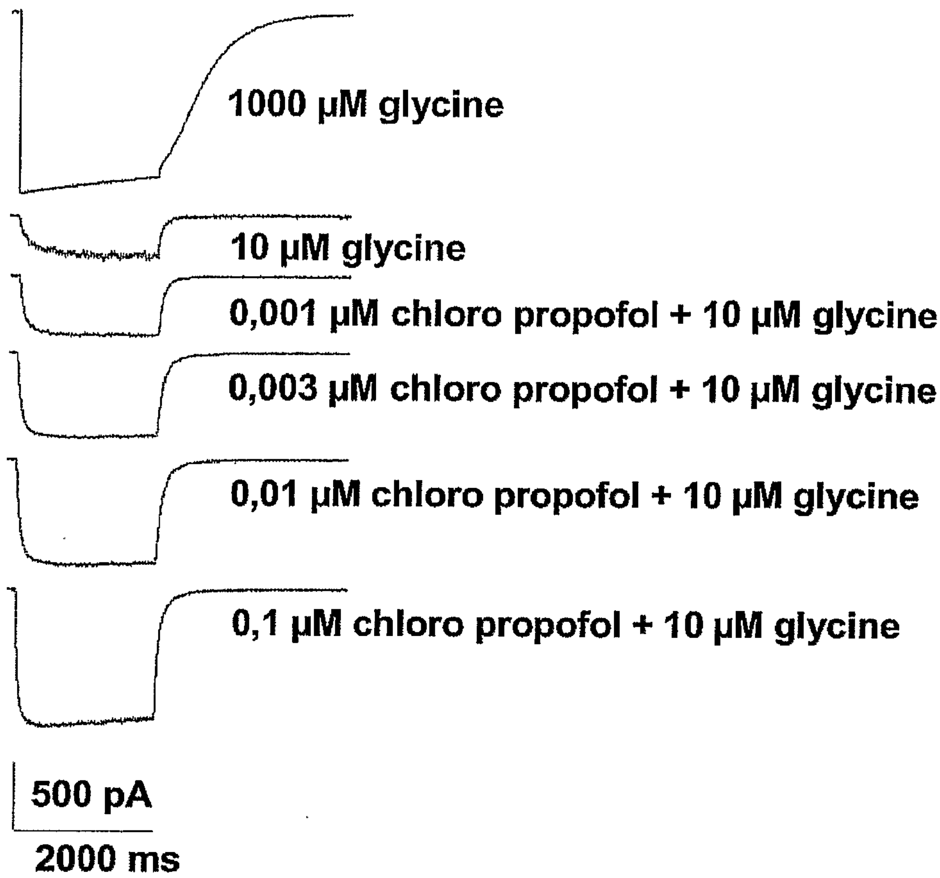
1000 µM glycine
10 µM glycine
0,001 µM chloro propofol + 10 µM glycine
0,003 µM chloro propofol + 10 µM glycine
0,01 µM chloro propofol + 10 µM glycine
0,1 µM chloro propofol + 10 µM glycine
500 pA
2000 ms

**FIG. 10**

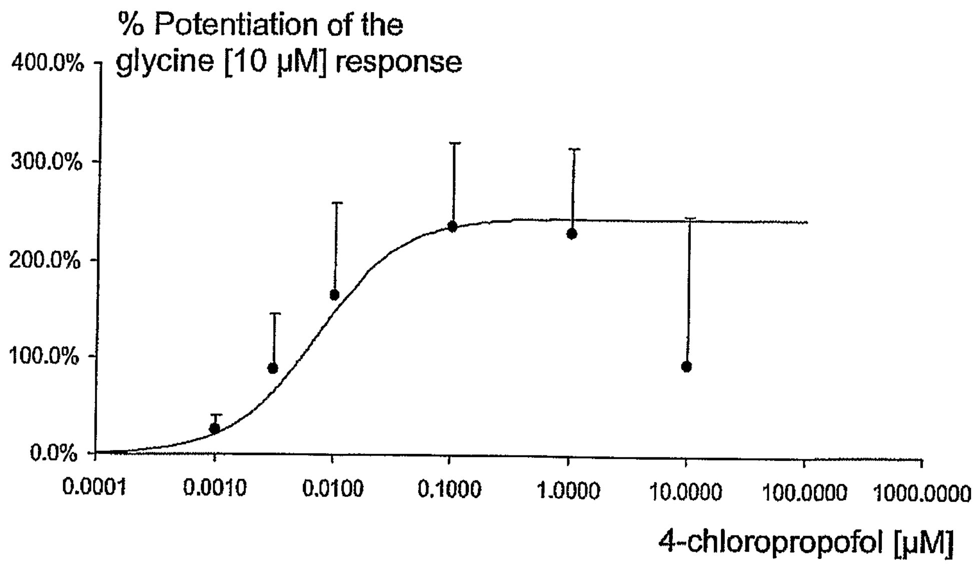
% Potentiation of the
glycine [10 μM] response
400.0%
300.0%
200.0%
100.0%
0.0%
0.0001
0.0010
0.0100
0.1000
1.0000
10.0000
100.0000
1000.0000
4-chloropropofol [μM]

**FIG. 11**

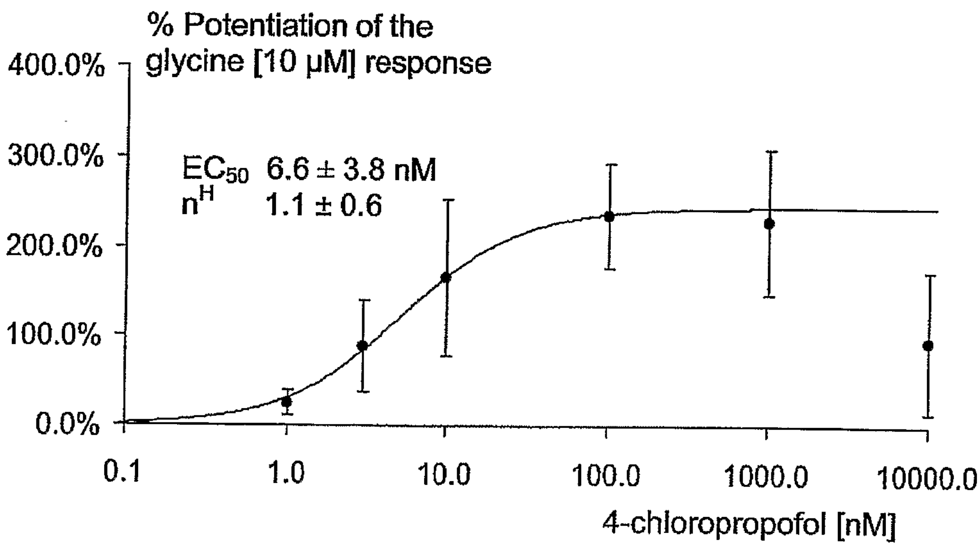
% Potentiation of the
glycine [10 μM] response
400.0%
300.0%
200.0%
100.0%
0.0%
EC50 6.6 ± 3.8 nM
nH 1.1 ± 0.6
0.1
1.0
10.0
100.0
1000.0
10000.0
4-chloropropofol [nM]

**FIG. 12**

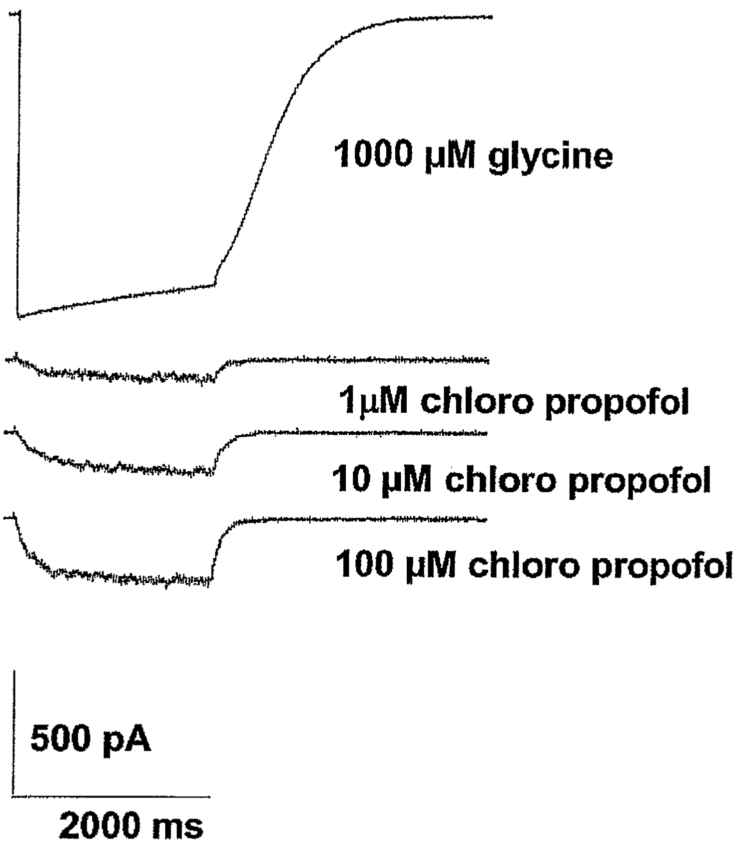
1000 µM glycine
1µM chloro propofol
10 µM chloro propofol
100 µM chloro propofol
500 pA
2000 ms

**FIG. 13**

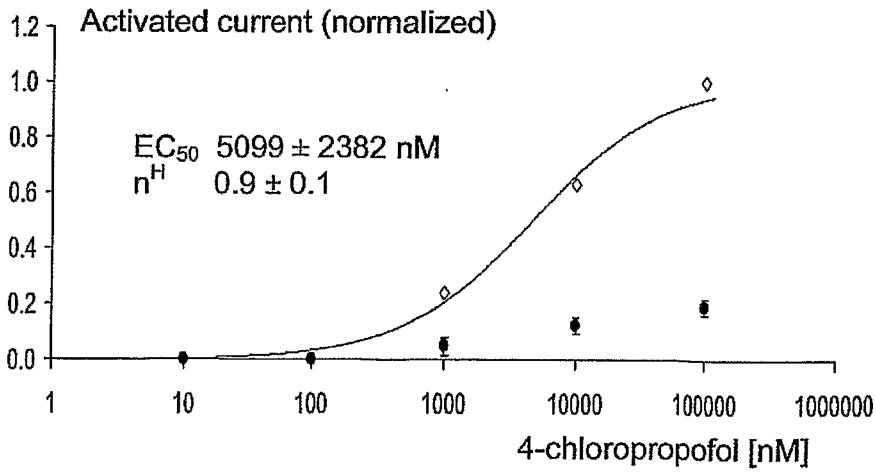
Activated current (normalized)
1.2
1.0
0.8
0.6
0.4
0.2
0.0
EC50 5099 ± 2382 nM
nH 0.9 ± 0.1
1
10
100
1000
10000
100000
1000000
4-chloropropofol [nM]

**FIG. 14**

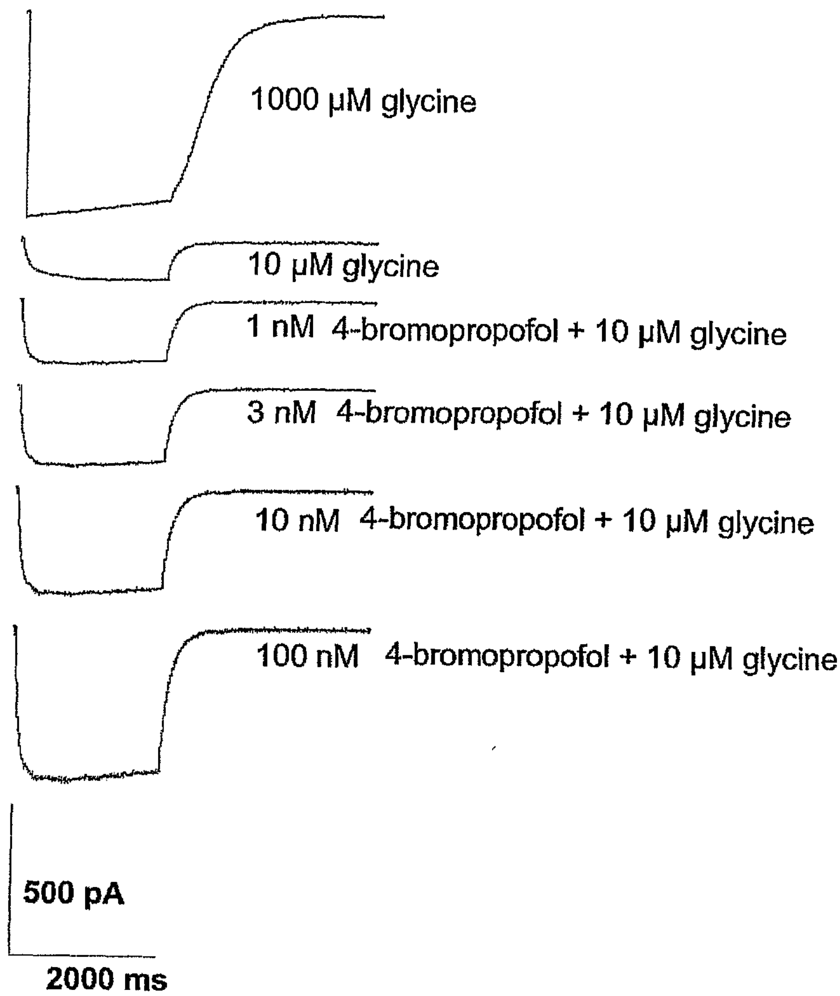
1000 µM glycine
10 µM glycine
1 nM 4-bromopropofol + 10 µM glycine
3 nM 4-bromopropofol + 10 µM glycine
10 nM 4-bromopropofol + 10 µM glycine
100 nM 4-bromopropofol + 10 µM glycine
500 pA
2000 ms

**FIG. 15**

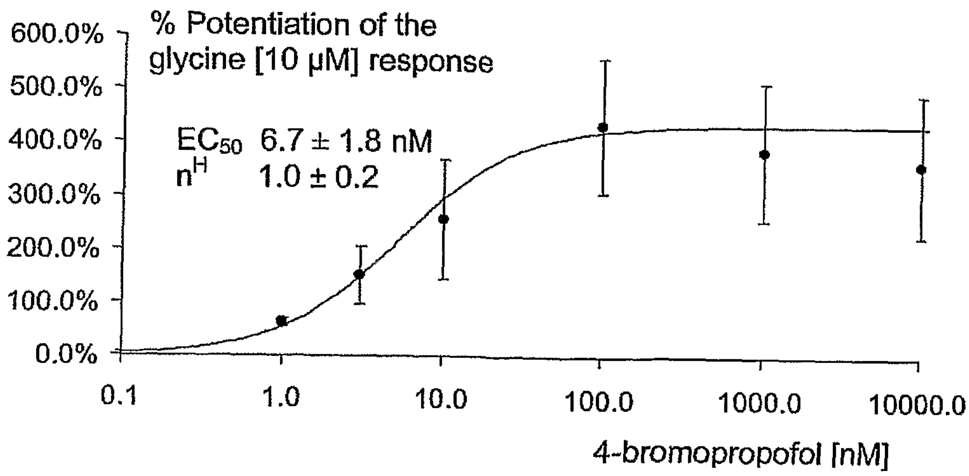
% Potentiation of the
glycine [10 µM] response
EC50 6.7 ± 1.8 nM
nH 1.0 ± 0.2
600.0%
500.0%
400.0%
300.0%
200.0%
100.0%
0.0%
0.1
1.0
10.0
100.0
1000.0
10000.0
4-bromopropofol [nM]

**FIG. 16**

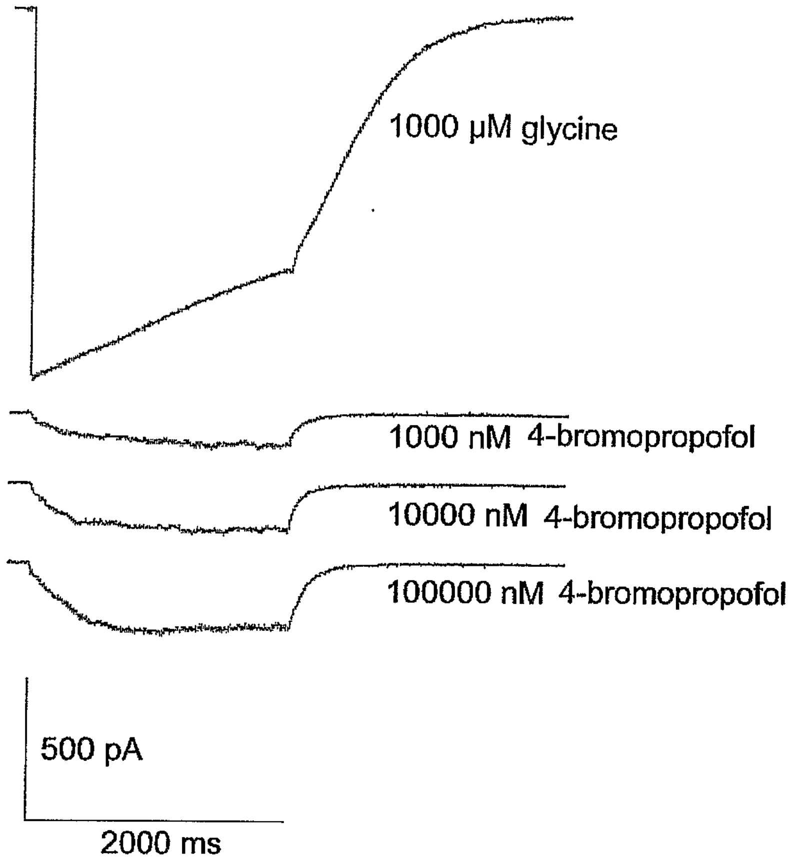
1000 µM glycine
1000 nM 4-bromopropofol
10000 nM 4-bromopropofol
100000 nM 4-bromopropofol
500 pA
2000 ms

**FIG. 17**

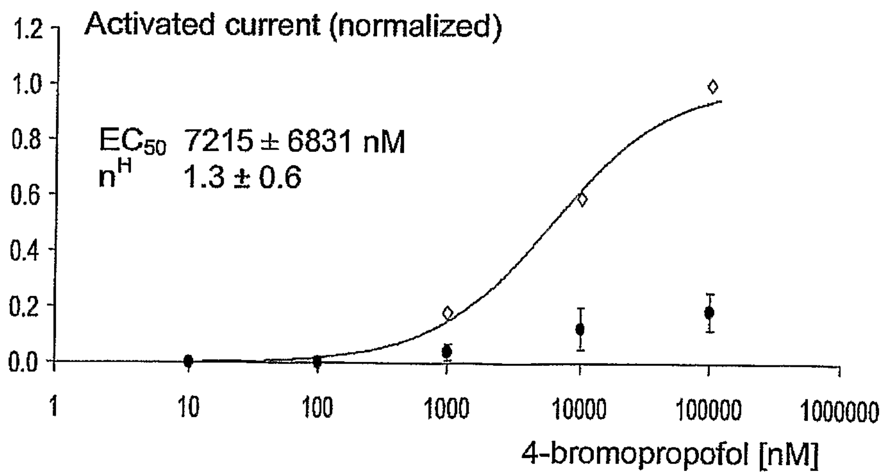
Activated current (normalized)
1.2
1.0
0.8
0.6
0.4
0.2
0.0
EC50 7215 ± 6831 nM
nH 1.3 ± 0.6
1
10
100
1000
10000
100000
1000000
4-bromopropofol [nM]

**FIG. 18**

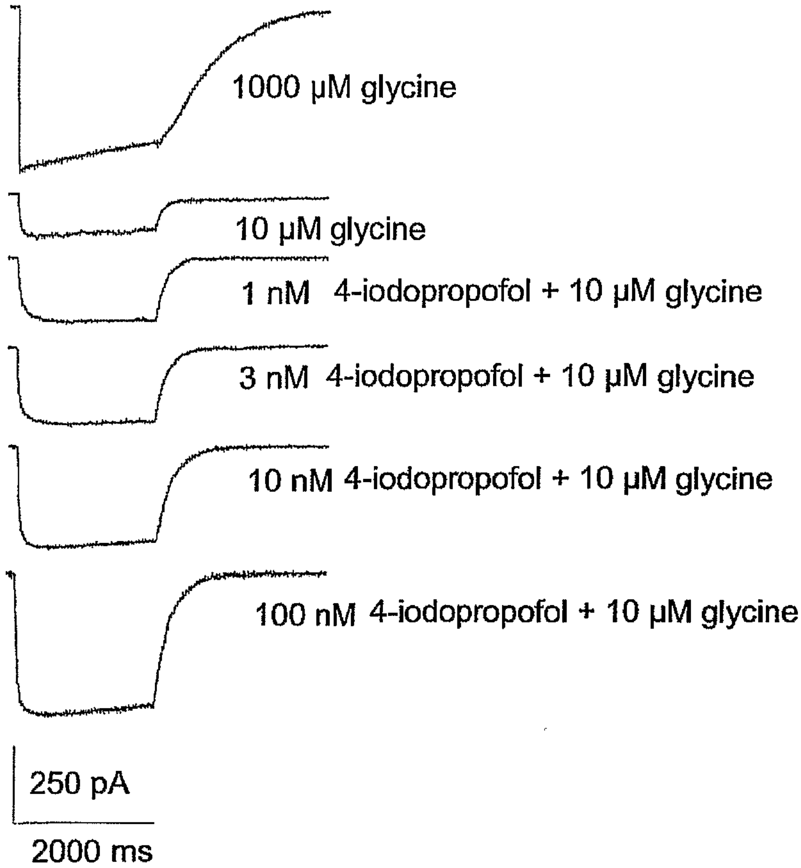
1000 µM glycine
10 µM glycine
1 nM 4-iodopropofol + 10 µM glycine
3 nM 4-iodopropofol + 10 µM glycine
10 nM 4-iodopropofol + 10 µM glycine
100 nM 4-iodopropofol + 10 µM glycine
250 pA
2000 ms

**FIG. 19**

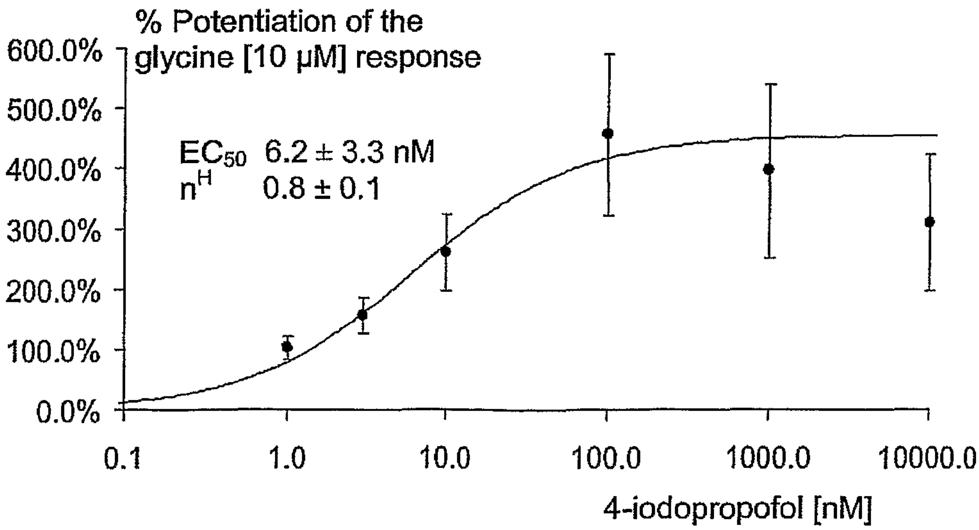
% Potentiation of the glycine [10 µM] response
600.0%
500.0%
400.0%
300.0%
200.0%
100.0%
0.0%
EC50 6.2 ± 3.3 nM
nH 0.8 ± 0.1
0.1
1.0
10.0
100.0
1000.0
10000.0
4-iodopropofol [nM]

**FIG. 20**

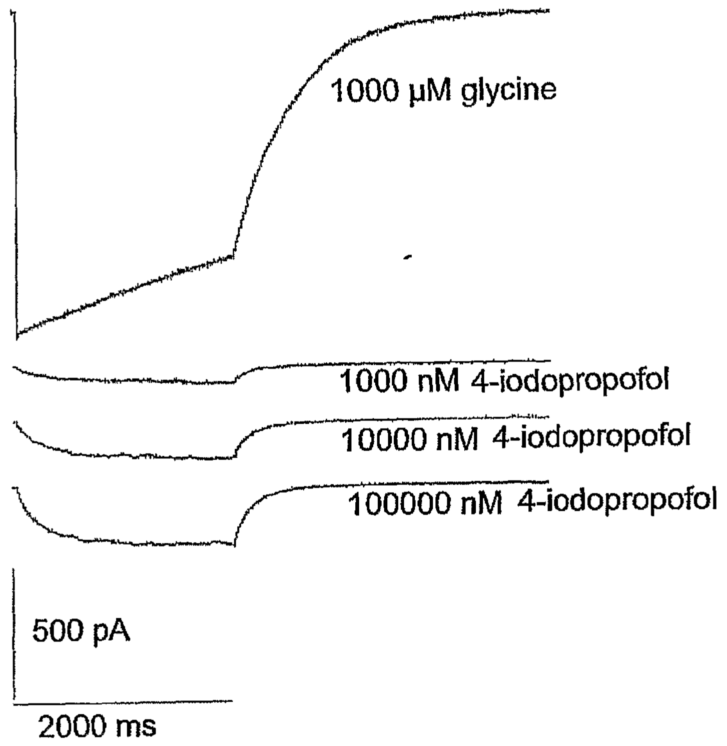
1000 µM glycine
1000 nM 4-iodopropofol
10000 nM 4-iodopropofol
100000 nM 4-iodopropofol
500 pA
2000 ms

**FIG. 21**

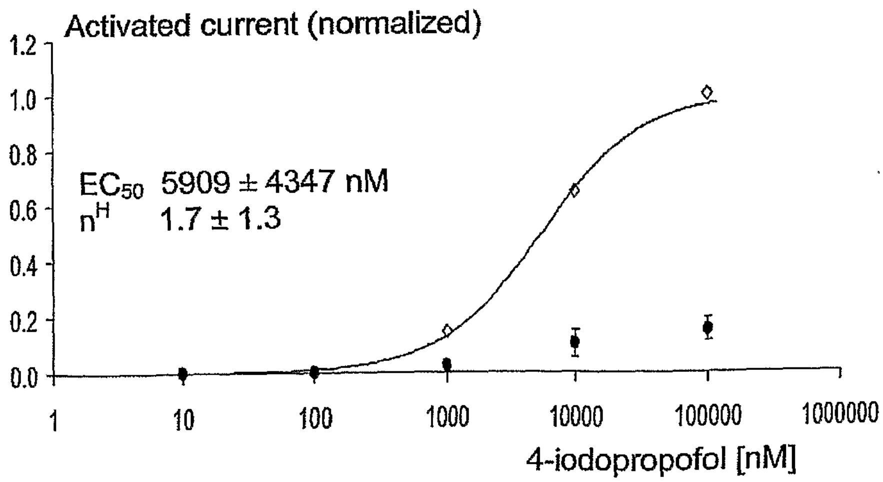
Activated current (normalized)
1.2
1.0
0.8
0.6
0.4
0.2
0.0
EC50 5909 ± 4347 nM
nH 1.7 ± 1.3
1
10
100
1000
10000
100000
1000000
4-iodopropofol [nM]

**FIG.22**

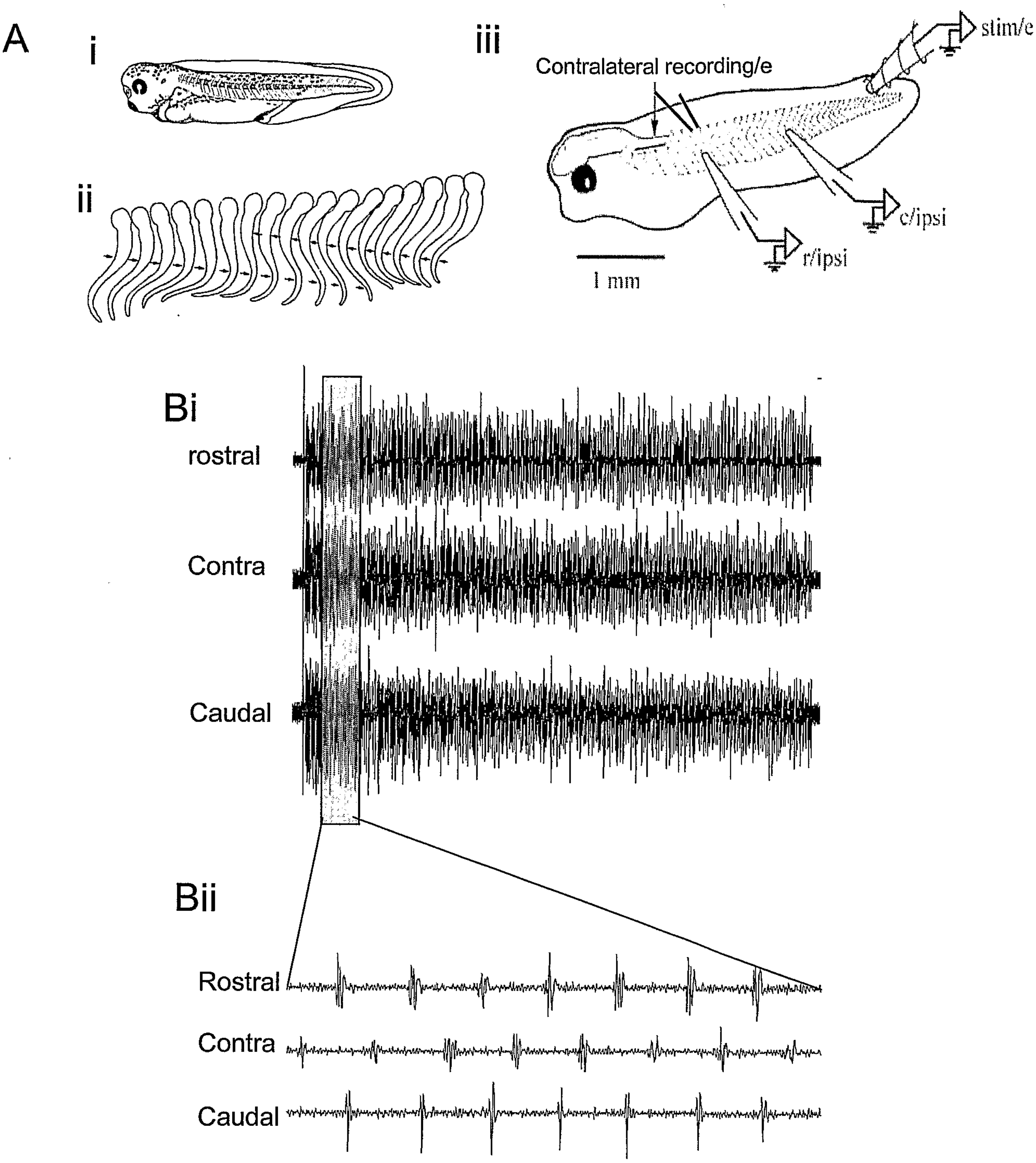
A
i
ii
iii
Contralateral recording/e
stim/e
c/ipsi
r/ipsi
1 mm
Bi
rostral
Contra
Caudal
Bii
Rostral
Contra
Caudal

FIG.23

Ai control – end of episode

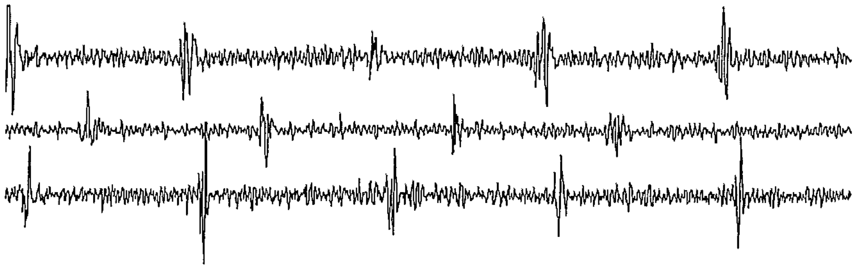

ii 10μM 4 chloropropofol – end of episode

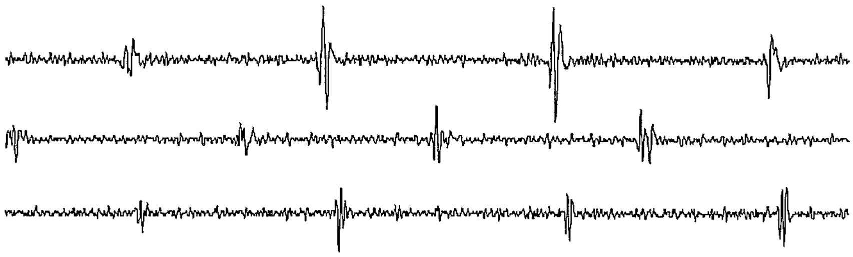

B

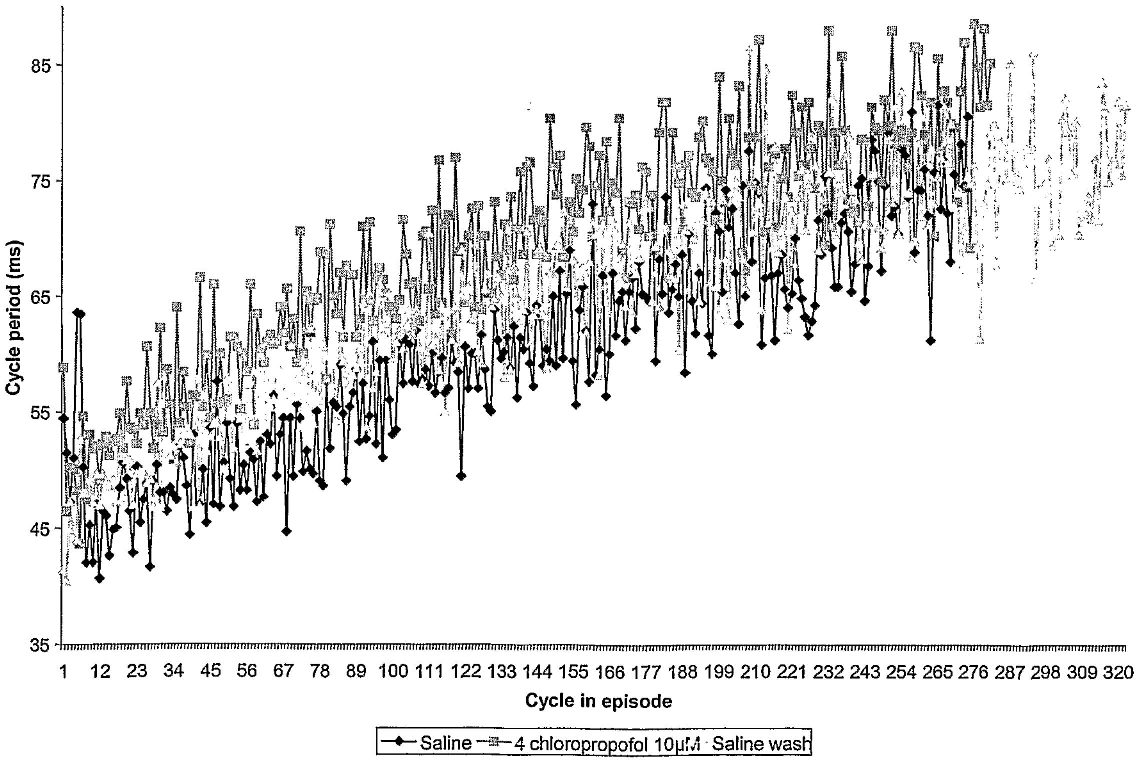

FIG.24

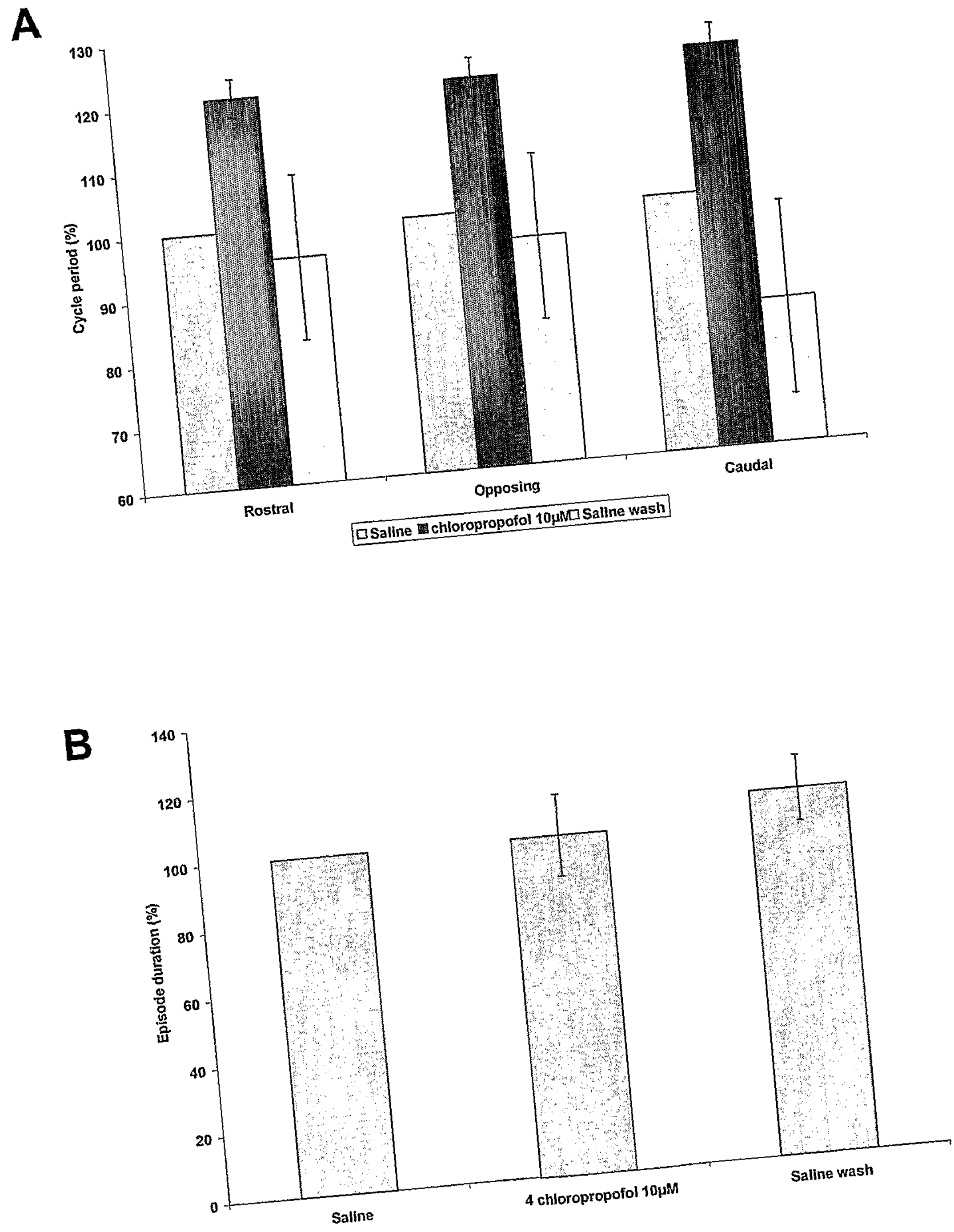
A
130
120
110
100
90
80
70
60
Cycle period (%)
Rostral
Opposing
Caudal
Saline
chloropropofol 10μM
Saline wash
B
140
120
100
80
60
40
20
0
Episode duration (%)
Saline
4 chloropropofol 10μM
Saline wash

FIG.25

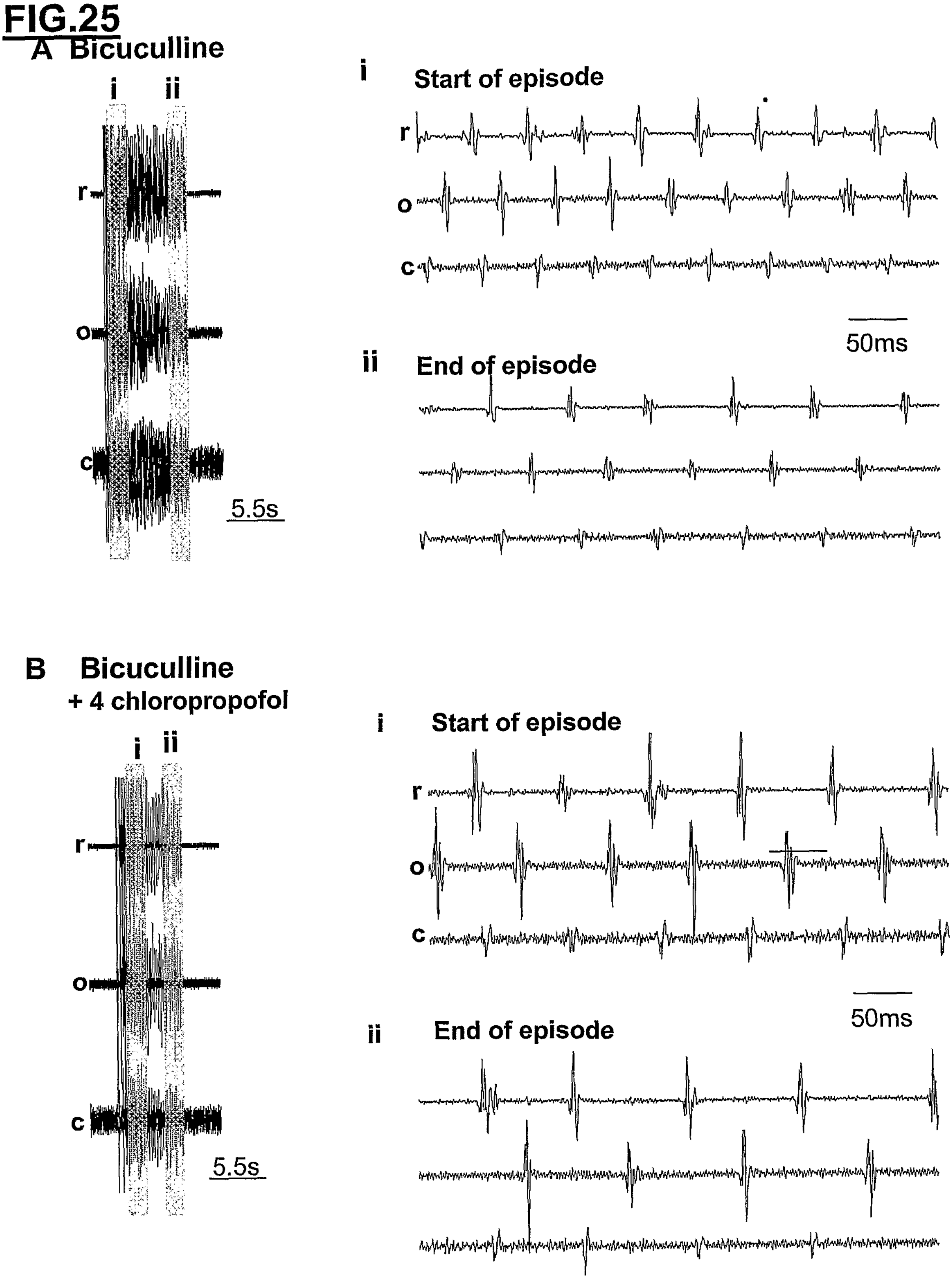
A Bicuculline
i
ii
r
o
c
5.5s
i Start of episode
r
o
c
50ms
ii End of episode
B Bicuculline
+ 4 chloropropofol
i
ii
r
o
c
5.5s
i Start of episode
r
o
c
50ms
ii End of episode

**FIG.26**

A

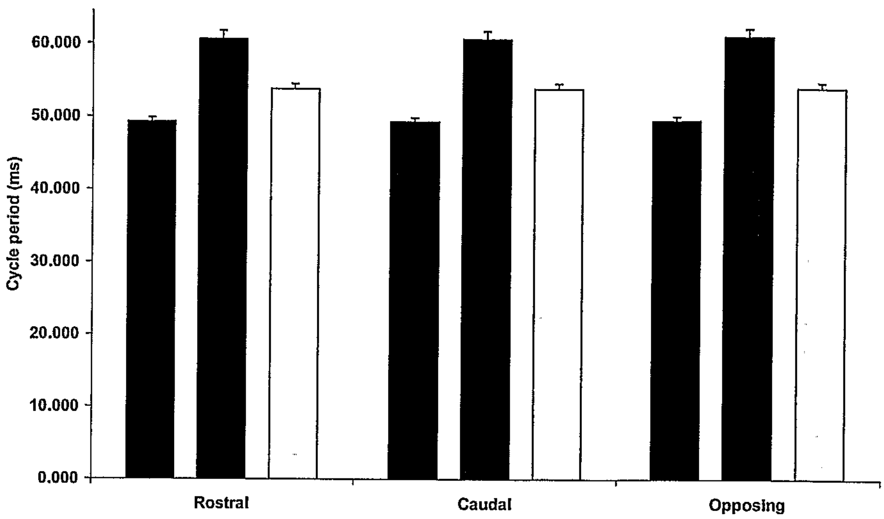
60.000
50.000
40.000
30.000
20.000
10.000
0.000
Cycle period (ms)
Rostral
Caudal
Opposing

B

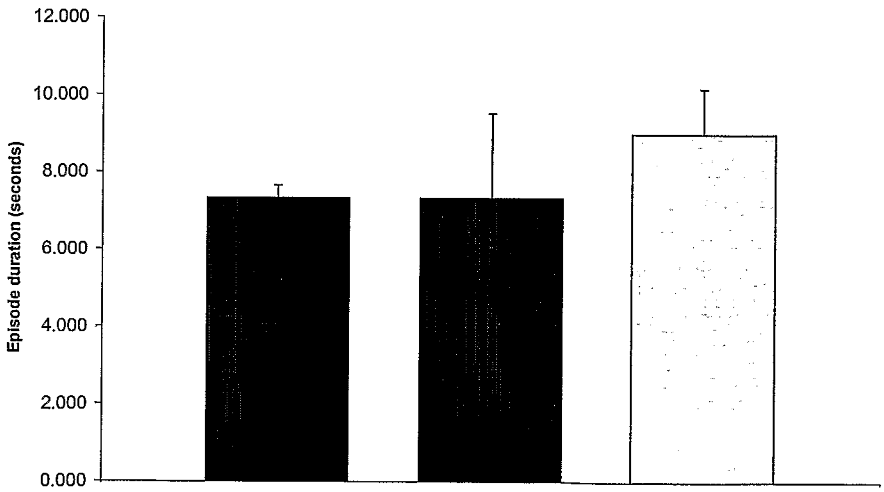
12.000
10.000
8.000
6.000
4.000
2.000
0.000
Episode duration (seconds)

**FIG.27**

Ai **Strychnine control - end of episode**

Aii **Strychnine + 4 chloropropofol - end of episode**

B

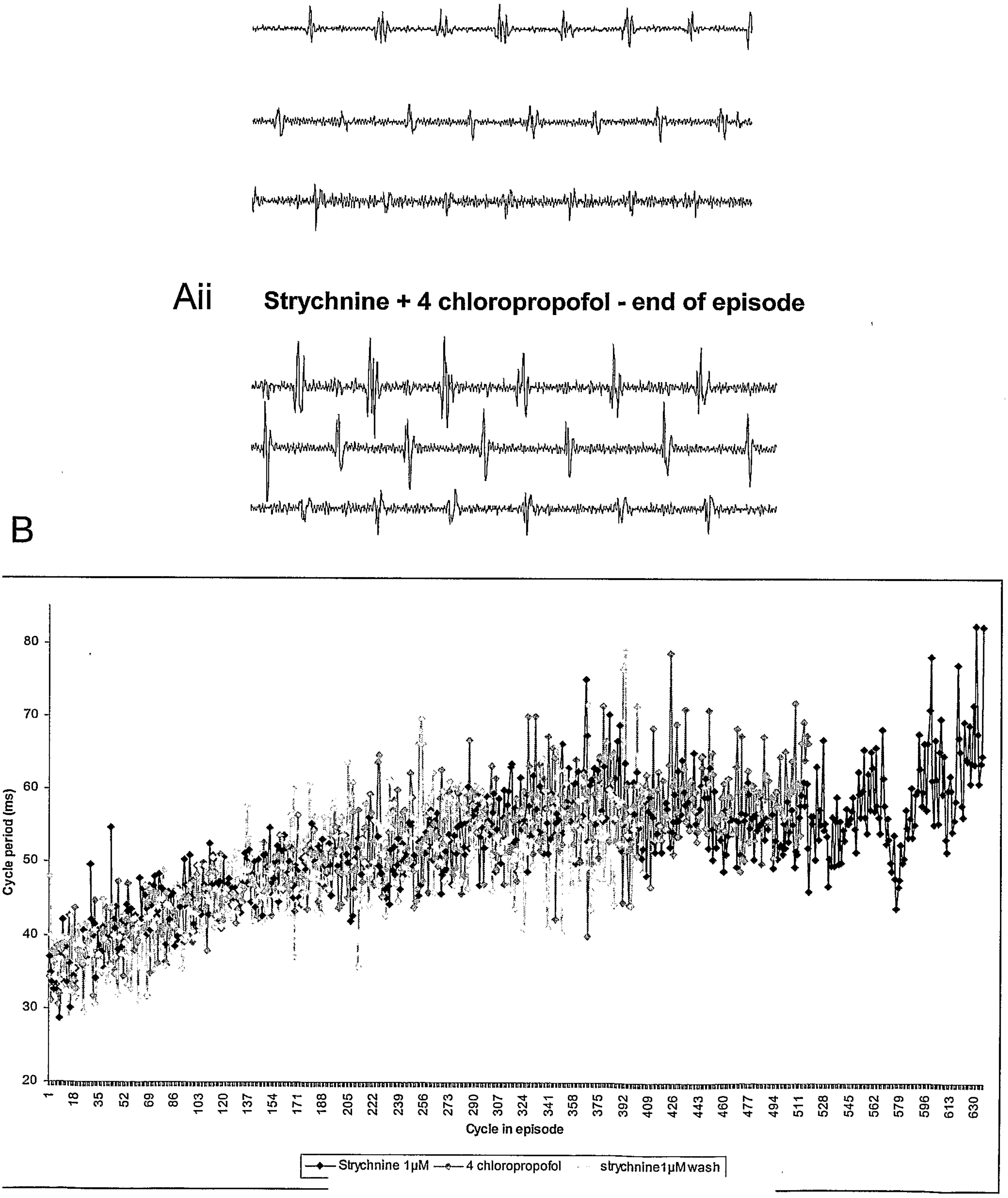

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0054588 A


### Non-patent literature cited in the description


- **AHMADI et al.** *Nature Neuroscience,* 2002, vol. 5 (1), 34-40
- **HAESELER et al.** *British Journal of Pharmacology,* 2005, vol. 145, 916-925
- **HAMILL et al.** *Pflügers Arch.,* 1981, vol. 391, 85-100
- **FRANKE et al.** *Neurosci. Lett.,* 1987, vol. 77, 199-204
- **RANDELL ; SELITTO.** *Arch Int Pharmacodyn,* 1957, vol. 4, 409-421